(19) Europäisches Patentamt / European Patent Office / Office européen des brevets

(11) **EP 3 658 919 B1**

(12) **EUROPEAN PATENT SPECIFICATION**

(45) Date of publication and mention of the grant of the patent:
**26.02.2025 Bulletin 2025/09**

(21) Application number: **18769818.8**

(22) Date of filing: **23.07.2018**

(51) International Patent Classification (IPC):
***G01N 33/74*** *(2006.01)*

(52) Cooperative Patent Classification (CPC):
**G01N 33/74;** G01N 2333/62; G01N 2800/52; G01N 2800/60

(86) International application number:
**PCT/PL2018/000077**

(87) International publication number:
**WO 2019/022628 (31.01.2019 Gazette 2019/05)**

(54) **A METHOD FOR DETERMINING TISSUE INSULIN SENSITIVITY, A METHOD FOR IDENTIFYING INSULIN RESISTANCE AND DETERMINING A PREDISPOSITION FOR A DISORDER CONNECTED THEREWITH AND USE OF INSULIN SENSITIVITY INDEX**

VERFAHREN ZUR BESTIMMUNG DER GEWEBEINSULINSENSITIVITÄT, VERFAHREN ZUR IDENTIFIZIERUNG DER INSULINRESISTENZ UND BESTIMMUNG EINER PRÄDISPOSITION FÜR EINE DAMIT VERBUNDENE ERKRANKUNG UND VERWENDUNG EINES INSULINEMPFINDLICHKEITSINDEX

PROCÉDÉ DE DÉTERMINATION DE SENSIBILITÉ À L'INSULINE DE TISSUS, PROCÉDÉ D'IDENTIFICATION DE RÉSISTANCE À L'INSULINE ET DE DÉTERMINATION D'UNE PRÉDISPOSITION À UN TROUBLE Y ÉTANT ASSOCIÉ ET UTILISATION D'INDICE DE SENSIBILITÉ À L'INSULINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2017 PL 42238817**

(43) Date of publication of application:
**03.06.2020 Bulletin 2020/23**

(73) Proprietor: **Uniwersytet Medyczny W Bialymstoku 15-089 Bialystok (PL)**

(72) Inventors:
• **KARCZEWSKA-KUPCZEWSKA, Monika 15-337 Bialystok (PL)**
• **STRACZKOWSKI, Marek 15-225 Bialystok (PL)**

(74) Representative: **Kawczynska, Marta Joanna Polservice Kancelaria Rzecznikow**

Patentowych sp. z o.o.
ul. Bluszczanska 73
00-712 Warszawa (PL)

(56) References cited:
• R. S. PATARRÃO ET AL: "Assessment of methods and indexes of insulin sensitivity", REV PORT ENDOCRINOL DIABETES METAB, vol. 9, no. 1, 1 January 2014 (2014-01-01), pages 65 - 73, XP055520898, DOI: 10.1016/j.rpedm.2013.10.004
• B. ANTUNA-PUENTE ET AL: "How can we measure insulin sensitivity/resistance?", DIABETES & METABOLISM, vol. 37, no. 3, 1 June 2011 (2011-06-01), pages 179 - 188, XP055520896, DOI: 10.1016/j.diabet.2011.01.002
• R. MUNIYAPPA ET AL: "Current approaches for assessing insulin sensitivity and resistance in vivo: advantages, limitations, and appropriate usage", AM J PHYSIOL ENDOCRINOL METAB, vol. 294, no. 1, 1 January 2008 (2008-01-01), pages E15 - E26, XP055003632, DOI: 10.1152/ajpendo.00645.2007

## Description

### Technical Field

The invention relates to an *in vitro* method for determining tissue insulin sensitivity in human subjects using the measurement of selected laboratory parameters and concentration of selected proteins in blood serum with the use of an insulin sensitivity index. The invention also relates to a method for identifying insulin resistance and determining a predisposition for a disorder connected with insulin resistance with the use of an insulin sensitivity index. Generally the use of an insulin sensitivity index as a diagnostic biomarker was herein described, and specifically the invention relates to the use of the insulin sensitivity index as an insulin sensitivity biomarker, a prognostic biomarker of a disorder connected with insulin resistance, in particular for the assessment of insulin resistance and a predisposition to disorders connected therewith, as well as for the diagnosis, monitoring and/or therapy monitoring of insulin resistance and a disorder connected therewith, such as overweight, obesity, impaired glucose tolerance, dyslipidaemia, metabolic syndrome, fatty liver, polycystic ovary syndrome, arterial hypertension, atherosclerosis, ischaemic heart disease, cerebral stroke, neurodegenerative disease, in particular type 2 diabetes. The invention also relates to the use of such insulin sensitivity index for monitoring tissue insulin sensitivity, in particular in the course of a disorder and/or a disease connected with insulin resistance, in particular type 2 diabetes.

### Background Art

Decreased response of an organism to insulin, i.e. insulin resistance, is the most important factor in the pathogenesis of type 2 diabetes. Insulin resistance is also connected with a predisposition for dyslipidaemia, arterial hypertension, atherosclerosis, ischaemic heart disease, neurodegenerative diseases and many other illnesses. Although a relationship between insulin resistance and obesity is known, reduced tissue insulin sensitivity may also occur in people with normal body weight. Reduced tissue insulin sensitivity in the case of many diseases and disorders has been described in literature (see, for example, Reaven GM. Banting lecture 1988. "Role of insulin resistance in human disease", Diabetes 1988, 37: 1595-1607). Insulin resistance and chronic inflammatory response connected therewith plays a role e.g. in the pathogenesis of atherosclerosis and cardiovascular diseases (see, for example, Fernandez-Real JM, Ricart W. "Insulin resistance and chronic cardiovascular inflammatory syndrome", Endocr Rev 2003, 24: 278-301, and in the publication of Nigro J, Osman N, Dart AM, Little PJ. Insulin resistance and atherosclerosis. Endocr Rev 2006, 27: 242-259). There has also been described the role of reduced insulin sensitivity in the brain aging process and cognitive impairment (see, for example, Roriz-Filho J, Sa-Roriz TM, Rosset I, Camozzato AL, Santos AC, Chaves ML, Moriguti JC, Roriz-Cruz M. (Pre) diabetes, brain aging, and cognition. Biochim Biophys Acta 2009, 1792: 432-443).

Early diagnosis of insulin resistance may therefore be essential for taking prophylactic measures in order to prevent diseases and disorders connected therewith. Methods for the assessment of tissue insulin sensitivity are known. There are available review articles concerning methods for the measurement of tissue insulin sensitivity.

At present, the "gold standard" among the methods for the *in vivo* assessment of tissue insulin sensitivity is represented by hyperinsulinemic-euglycemic clamp technique, which was introduced by DeFronzo et al. This method was described in detail in an article by DeFronzo RA, Tobin JD, Andres R. "Glucose clamp technique: a method for quantifying insulin secretion and resistance.", Am J Physiol 1979, 237: E214-E223. Since the time of publishing the article, the clamp technique has been widely used in studies, including tests in people; however, the practical application of this technique is limited to scientific research carried out in specialized centres. A clamp technique test requires two venipunctures, at least 2-hour continuous infusion of insulin and variable, controlled infusion of glucose, and performing blood draw every 5 minutes. It requires using specialized medical equipment, employing qualified medical personnel and is troublesome and inconvenient for the person subjected to the test.

The available literature concerning methods for the assessment of tissue insulin sensitivity highlights limitations connected with tests by the clamp technique, as well as other methods used for this purpose, such as insulin suppression test which requires 3-hours infusion of glucose, insulin and samatostatin or its analogue - octreotide (see, for example, Muniyappa R, Madan R, Quon MJ. "Assessing insulin sensitivity and resistance in humans", in: De Groot LJ, Chrousos G, Dungan K, Feingold KR, Grossman A, Hershman JM, Koch C, Korbonits M, McLachlan R, New M, Pumell J, Rebar R, Singer F, Vinik A, wyd. Endotext [Internet]. South Dartmouth (MA): MDText.com, Inc.; 2000-. ; Antuna-Puente B, Disse E, Rabasa-Lhoret R, Laville M, Capeau J, Bastard JP. "How can we measure insulin sensitivity/resistance?", Diabetes Metab 2011, 37: 179-188; 9; Wallace TM, Matthews DR., "The assessment of insulin resistance in man.", Diabet Med 2002, 19: 527-534; Ferrannini E, Mari A. "How to measure insulin sensitivity." J Hypertens 1998, 16:895-906; Patarrao RS et al. "Assessment of methods and indexes of insulin sensitivity", Rev Port Endocrinol Diabetes Metab 2014, 9(1): 65-73; Muniyappa R et al. "Current approaches for assessing insulin sensitivity and resistance in vivo: advantages, limitations, and appropriate usage", Am J Physiol Endocrinol Metab 2008, 294(1): E15-E26).

The limitations of currently available diagnostic methods described in the literature are primarily connected with the fact that these tests are time-consuming, laborious and expensive, and they require specialized equipment and qualified personnel. That is why, neither the clamp technique nor the insulin suppression test are deemed to be capable of application in epidemiological studies, large clinical trials and in everyday medical practice.

There have also been described other dynamic tests for the assessment of tissue insulin sensitivity, such as the intravenous glucose tolerance test with minimal model analysis. However, it also requires intravenous infusion and multiple blood draws. Additionally, while being almost equally laborious, it is not as precise as the clamp technique.

The known methods of determining tissue insulin sensitivity include indices based on the oral glucose tolerance test. It is a 2-hour, standard 75-gram glucose load test used in clinical practice, in which glucose tolerance is assessed. One exemplary tissue insulin sensitivity index developed on the basis of the oral glucose tolerance test is the Matsuda Index (Matsuda M. "Measuring and estimating insulin resistance in clinical research setting.", Nutr Metab Cardiovasc Dis 2010, 20: 79-86). To calculate this index, it is necessary to determine the concentration of glucose and insulin in blood in 0, $30^{th}$, $60^{th}$, $90^{th}$ and 120th minute of the glucose tolerance test, which is rather time-consuming and requires multiple blood draws. It must be emphasized that the glucose tolerance is affected by not only tissue insulin sensitivity but also insulin secretion, insulin absorption rate, incretin effect, suppression of glucagon level and many other factors. Oral glucose tolerance test shows also low reproducibility (see, for example, Ko GT, Chan JC, Woo J, Lau E, Yeung VT, Chow CC, Cockram CS. "The reproducibility and usefulness of the oral glucose tolerance test in screening for diabetes and other cardiovascular risk factors." Ann Clin Biochem 1998; 35: 62-67; Libman IM, Barinas-Mitchell E, Bartucci A, Robertson R, Arslanian S. "Reproducibility of the Oral Glucose Tolerance Test in Overweight Children." J Clin Endocrinol Metab 2008; 93: 4231-4237). The measurement of insulin sensitivity based on the oral glucose tolerance has failed to find application in general population or diagnostic and epidemiological studies, or in everyday medical practice.

There have also been developed simple indices reflecting tissue insulin sensitivity, such as HOMA-IR (Homeostasis Model Assessment - Insulin Resistance) or QUICKI (Quantitative Insulin Sensitivity Check Index), involving the measurement of fasting blood glucose and insulin concentrations. These indices also have some limitations resulting, among others, from the fact that they primarily reflect hepatic insulin sensitivity/resistance, whereas, actually, in the predisposition/for the development of a disorder connected with reduced tissue insulin sensitivity, such as type 2 diabetes, the most import role is played by peripheral insulin resistance, for which skeletal muscles are primarily responsible (and which is measured in the test using clamp technique). It should be emphasized that insulin is secreted in a pulsatile manner and glucose concentration in blood is controlled not only by insulin but also by many other hormones, e.g., glucagon. Individual fluctuations in insulinaemia are also affected by many other factors, e.g., stress or physical activity. Although such indices as HOMA-IR and QUICKI generally show good correlation with results obtained using the clamp technique, when human subjects with a marked insulin resistance and/or glucose intolerance are taken into account, this relation is considerably weaker in general population. For these reasons HOMA-IR and QUICKI indices have some limitations in identifying human subjects with insulin resistance in general population (Ruige JB, Mertens IL, Bartholomeeusen E, Dirinck E, Ferrannini E, Van Gaal LF., "Fasting-based estimates of insulin sensitivity in overweight and obesity: a critical appraisal.", Obesity 2006, 14: 1250-1256; Malita FM, Karelis AD, St-Pierre DH, Garrel D, Bastard JP, Tardif A, Prud'homme D, Rabasa-Lhoret R., "Surrogate indexes vs. euglycaemic-hyperinsulinemic clamp as an indicator of insulin resistance and cardiovascular risk factors in overweight and obese postmenopausal women.", Diabetes Metab 2006, 32: 251-255).

Considering the fact that dynamic tests are complicated, time-consuming and laborious, hardly reproducible, and indices based on the determination of fasting blood glucose and insulin are excessively simplified, and to a lesser extent assess systemic insulin resistance, at present, there is no ideal simple index assessing tissue insulin sensitivity in clinical and population studies and in everyday medical practice.

## Object of the Invention

The object of the present invention is to provide simple and reliable means and diagnostic methods which reflect tissue insulin sensitivity and enable the identification, early diagnosis and/or detection of insulin resistance and a predisposition for diseases connected with reduced tissue insulin sensitivity, i.e. insulin resistance, in general population, and which simultaneously ensure repeatable results with high sensitivity and specificity. The object of the present invention is therefore to provide new, effective tools for determining tissue insulin sensitivity, ensuring repeatable results with high sensitivity and specificity, in a quick, reliable and cost-effective way, suitable for routine applications in general population, in epidemiological studies, large clinical trials and in everyday medical practice, including the assessment of tissue insulin sensitivity, identification of insulin resistance and determination of a predisposition for disorders connected with insulin resistance, early diagnosis of diseases connected with insulin resistance, as well as monitoring of the course of the treatment and clinical assessment of the progression of such disorders. The purpose of the present invention is also to provide new, effective tools for *in vitro* determining tissue insulin sensitivity and identifying insulin resistance in a manner that is convenient for the patient, which minimizes his discomfort and pain connected with long-lasting testing, multiple insertions of needles and multiple blood draws. The above objects have been realized by the invention defined in the patent claims,

## Summary of the Invention

The invention is set out in the appended set of patent claims.

The subject matter of the present invention relates to an *in vitro* method for determining tissue insulin sensitivity in a human subject, consisting in that

a) in a blood sample drawn from a human subject, the following laboratory parameters and concentrations of the following proteins in blood serum are measured: red blood cells (RBC), alanine aminotransferase (AlAT), C-peptide, sex hormone binding globulin (SHBG), adiponectin, insulin-like growth factor binding protein-1 (IGFBP1);

b) an insulin sensitivity index is determined on the basis of obtained results of measurements specified in a) using the following equation:

Insulin sensitivity index = 11.5847 - RBC x 0.9622 - AlAT x 0.0308 - C-peptide x 1.0718 + SHBG x 0.0239 + Adiponectin x 0.0466 + IGFBP1 x 0.1206,

wherein

RBC is the value of red blood cells count measured in the sample using any method having 4-6 mln/$\mu$l reference interval for general population;

AlAT is the value of alanine aminotransferase activity measured in the sample using any method having 5 - 50 U/l reference interval for general population;

C-peptide is the value of C-peptide concentration measured using any method having 0.003 - 0.04 pmol/ml sensitivity and 0.26 - 2 pmol/ml reference interval for general population;

SHBG is the value of the concentration of sex hormone-binding globulin measured using any method having 0.02 - 0.35 nmol/l sensitivity and 9 - 144 nmol/l reference interval for general population;

Adiponectin is the value of adiponectin concentration measured using any method having 0.15 - 0.5 $\mu$g/ml sensitivity and 6 - 30 $\mu$g/ml reference interval for general population;

IGFBP1 is the value of the concentration of insulin-like growth factor binding protein-1 measured using any method having 0.009 - 0.02 ng/ml sensitivity and 0.2 - 18 ng/ml reference interval for general population.

The subject matter of the invention thus relates also to a method for identifying insulin resistance and determining a predisposition for a disorder connected with insulin resistance in a human subject, consisting in that

a) in a blood sample drawn from a human subject, the following laboratory parameters and concentrations of the following proteins in blood serum are measured: red blood cells (RBC), alanine aminotransferase (AlAT), C-peptide, sex hormone binding globulin (SHBG), adiponectin, insulin-like growth factor binding protein-1 (IGFBP1);

b) an insulin sensitivity index is determined on the basis of obtained results of measurements specified in a) using the following equation:

Insulin sensitivity index = 11.5847 - RBC x 0.9622 - AlAT x 0.0308 - C-peptide x 1.0718 + SHBG x 0.0239 + Adiponectin x 0.0466 + IGFBP1 x 0.1206,

wherein

RBC is the value of red blood cells count measured in the sample using any method having 4-6 mln/$\mu$l reference interval for general population;

AlAT is the value of alanine aminotransferase activity measured in the sample using any method having 5 - 50 U/l reference interval for general population;

C-peptide is the value of C-peptide concentration measured using any method having 0.003 - 0.04 pmol/ml sensitivity and 0.26 - 2 pmol/ml reference interval for general population;

SHBG is the value of the concentration of sex hormone-binding globulin measured using any method having 0.02 - 0.35 nmol/l sensitivity and 9-144 nmol/l reference interval for general population;

Adiponectin is the value of adiponectin concentration measured using any method having 0.15 - 0.5 $\mu$g/ml sensitivity and 6 - 30 $\mu$g/ml reference interval for general population;

IGFBP1 is the value of the concentration of insulin-like growth factor binding protein-1 measured using any method having 0.009 - 0.02 ng/ml sensitivity and 0.2 - 18 ng/ml reference interval for general population;

c) insulin resistance is identified and a predisposition for a disorder connected with insulin resistance is determined when obtained value of insulin sensitivity index is 7.5 or less.

In the methods according to the invention RBC count is preferably measured using a cytometry method with optical laser light scattering analysis and/or an impedance method, AlAT activity is measured using a kinetic spectrophotometric method or a calorimetric method, C-peptide concentration is measured using ELISA, RIA method or a chemiluminescence method, SHBG concentration is measured using IRMA, ELISA or a chemiluminescence method, adiponectin concentration is measured using RIA or ELISA method, and IGFBP1 concentration is measured using ELISA or RIA method.

More preferably, in the methods according to the invention RBC count is measured using a cytometry method with optical laser light scattering analysis, AlAT activity is measured using a kinetic spectrophotometric method, C-peptide and IGFBP1 concentrations are measured using ELISA method, SHBG concentration is measured using IRMA method, and adiponectin concentration is measured using RIA method.

The methods according to the invention are preferably used for the prognosis, diagnosis and/or therapy monitoring of a disorder connected with insulin resistance.

Preferably, the methods according to the invention are used for the prognosis, diagnosis and/or therapy monitoring of a disorder connected with insulin resistance selected from a group comprising: overweight, obesity, impaired glucose tolerance, dyslipidaemia, metabolic syndrome, fatty liver, polycystic ovary syndrome, arterial hypertension, atherosclerosis, ischaemic heart disease, cerebral stroke, neurodegenerative diseases, in particular type 2 diabetes.

Preferably, in the methods according to the invention a blood sample is derived from a human subject selected from a group comprising: a person with normal body weight, an overweight person, an obese person, a person with increased fasting glycaemia, a person with impaired glucose tolerance, a person with type 2 diabetes, a person with dyslipidaemia, a person with metabolic syndrome, a person with fatty liver, a woman with polycystic ovary syndrome, a person with arterial hypertension, a person with atherosclerosis, a person with ischaemic heart disease, a person after cerebral stroke, a person with a neurodegenerative disease, a woman who had gestational diabetes or gave birth to a baby having over 4 kg body weight, and a first-degree relative of a person suffering from diabetes and/or cardio-vascular diseases, and a person before bariatric surgery and/or after such surgery.

The subject matter of the invention also relates to the use of an insulin sensitivity index defined by the following equation:

Insulin sensitivity index = 11.5847 - RBC x 0.9622 - AlAT x 0.0308 - C-peptide x 1.0718 + SHBG x 0.0239 + Adiponectin x 0.0466 + IGFBP1 x 0.1206

wherein

RBC is the value of red blood cells count measured in the sample using any method having 4-6 mln/$\mu$l reference interval for general population;
AlAT is the value of alanine aminotransferase activity measured in the sample using any method having 5 - 50 U/l reference interval for general population;
C-peptide is the value of C-peptide concentration measured using any method having 0.003 - 0.04 pmol/ml sensitivity and 0.26 - 2 pmol/ml reference interval for general population;
SHBG is the value of the concentration of sex hormone-binding globulin measured using any method having 0.02 - 0.35 nmol/l sensitivity and 9 - 144 nmol/l reference interval for general population;
Adiponectin is the value of adiponectin concentration measured using any method having 0.15 - 0.5 $\mu$g/ml sensitivity and 6 - 30 $\mu$g/ml reference interval for general population;
IGFBP1 is the value of the concentration of insulin-like growth factor binding protein-1 measured using any method having 0.009 - 0.02 ng/ml sensitivity and 0.2 - 18 ng/ml reference interval for general population;
as an insulin resistance biomarker.

The subject matter of the invention relates to the use of the above-defined insulin sensitivity index as a biomarker of insulin resistance, i.e. reduced tissue insulin sensitivity.

The subject matter of the invention also relates to the use of the above-defined insulin sensitivity index as a prognostic biomarker of a disorder connected with insulin resistance.

The subject matter of the invention further relates to the use of the above-defined insulin sensitivity index for determining a predisposition for a disorder connected with insulin resistance, i.e. reduced tissue insulin sensitivity.

Preferably, in the case of the use of insulin sensitivity index according to the invention RBC count is measured using a cytometry method with optical laser light scattering analysis and/or an impedance method, AlAT activity is measured using a kinetic spectrophotometric method or a calorimetric method, C-peptide concentration is measured using ELISA, RIA method or a chemiluminescence method, SHBG concentration is measured using IRMA, ELISA or a chemiluminescence method, adiponectin concentration is measured using RIA or ELISA method, and IGFBP1 concentration is measured using ELISA or RIA method.

More preferably, RBC count is measured using a cytometry method with optical laser light scattering analysis, AIAT activity is measured using a kinetic spectrophotometric method, C-peptide and IGFBP1 concentration is measured using ELISA method, SHBG concentration is measured using IRMA method, and adiponectin concentration is measured using RIA method.

Preferably, the use of the above-defined insulin sensitivity index according to the invention is further for the prognosis, diagnosis and/or monitoring of a disorder connected with insulin resistance selected from a group comprising: overweight, obesity, impaired glucose tolerance, dyslipidaemia, metabolic syndrome, polycystic ovary syndrome, fatty liver, arterial hypertension, atherosclerosis, ischaemic heart disease, cerebral stroke, and in particular type 2 diabetes.

More preferably, the use of the above-defined insulin sensitivity index according to the invention is further for the prognosis, diagnosis and/or monitoring of a disorder connected with insulin resistance in a human subject selected from a group comprising: a person with normal body weight, an overweight person, an obese person, a person with increased fasting glycaemia, a person with impaired glucose tolerance, a person with type 2 diabetes, a person with dyslipidaemia, a person with metabolic syndrome, a person with fatty liver, a woman with polycystic ovary syndrome, a person with arterial hypertension, a person with atherosclerosis, a person with ischaemic heart disease, a person after cerebral stroke, a woman who had gestational diabetes or gave birth to a baby having over 4 kg body weight, and a first-degree relative of a person suffering from diabetes and/or cardio-vascular diseases, and a person before bariatric surgery and/or after such surgery.

The subject matter of the invention also relates to the use of an insulin sensitivity index defined by the following equation:

Insulin sensitivity index = 11.5847 - RBC x 0.9622 - AIAT x 0.0308 - C-peptide x 1.0718 + SHBG x 0.0239 + Adiponectin x 0.0466 + IGFBP1 x 0.1206

wherein

RBC is the value of red blood cells count measured in the sample using any method having 4-6 mln/$\mu$l reference interval for general population;

AIAT is the value of alanine aminotransferase activity measured in the sample using any method having 5 - 50 U/l reference interval for general population;

C-peptide is the value of C-peptide concentration measured using any method having 0.003 - 0.04 pmol/ml sensitivity and 0.26 - 2 pmol/ml reference interval for general population;

SHBG is the value of the concentration of sex hormone-binding globulin measured using any method having 0.02 - 0.35 nmol/l sensitivity and 9 - 144 nmol/l reference interval for general population;

Adiponectin is the value of adiponectin concentration measured using any method having 0.15 - 0.5 $\mu$g/ml sensitivity and 6 - 30 $\mu$g/ml reference interval for general population;

IGFBP1 is the value of the concentration of insulin-like growth factor binding protein-1 measured using any method having 0.009 - 0.02 ng/ml sensitivity and 0.2 - 18 ng/ml reference interval for general population;

for monitoring tissue insulin sensitivity, in particular in a disorder and/or a disease connected with insulin resistance, in particular type 2 diabetes.

Preferably, in such use according to the invention RBC count is measured using a cytometry method with optical laser light scattering analysis and/or an impedance method, AIAT activity is measured using a kinetic spectrophotometric method or a calorimetric method, C-peptide concentration is measured using ELISA, RIA method or a chemiluminescence method, SHBG concentration is measured using IRMA, ELISA or a chemiluminescence method, adiponectin concentration is measured using RIA or ELISA method, and IGFBP1 concentration is measured using ELISA or RIA method.

More preferably, in such use according to the invention RBC count is measured using a cytometry method with optical laser light scattering analysis, AIAT activity is measured using a kinetic spectrophotometric method, C-peptide and IGFBP1 concentrations are measured using ELISA method, SHBG concentration is measured using IRMA method, and adiponectin concentration is measured using RIA method.

Preferably, such use according to the invention is applied for the monitoring of tissue insulin sensitivity in a disorder connected with insulin resistance selected from a group comprising: overweight, obesity, impaired glucose tolerance, dyslipidaemia, metabolic syndrome, polycystic ovary syndrome, fatty liver, arterial hypertension, atherosclerosis, ischaemic heart disease, cerebral stroke, neurodegenerative diseases, and in particular type 2 diabetes.

Preferably, such use according to the invention is applied for the monitoring of tissue insulin sensitivity in a human subject selected from a group comprising: a person with normal body weight, an overweight person, an obese person, a person with increased fasting glycaemia, a person with impaired glucose tolerance, a person with type 2 diabetes, a person with dyslipidaemia, a person with metabolic syndrome, a person with fatty liver, a woman with polycystic ovary syndrome, a person with arterial hypertension, a person with atherosclerosis, a person with ischaemic heart disease, a person after cerebral stroke, a person with a neurodegenerative disease, a woman who had gestational diabetes or gave birth to a baby having over 4 kg body weight, and a first-degree relative of a person suffering from diabetes and/or cardio-vascular diseases, and a person before bariatric surgery and/or after such surgery.

**Detailed Description of the Invention**

The present inventors have developed a new *in vitro* method for determining tissue insulin sensitivity using the measurement of selected laboratory parameters in a sample obtained from one blood draw from a human subject and of the concentration of specific proteins in serum obtained therefrom. More specifically, the method for determining tissue insulin sensitivity according to the invention requires the measurement of red blood cells (RBC) count, the measurement of alanine aminotransferase (AlAT) activity and the serum concentration of C-peptide, SHBG (sex hormone-binding globulin), adiponectin and IGFBP-1 (insulin-like growth factor binding protein-1) in a blood sample obtained from one blood draw from the tested human subject. For this purpose it is required to use any standard, commonly known methods and devices, commonly known and used in the field of procedures for the preparation of biological material, and measurements of specific parameters in whole blood or in serum. The results of the above measurements are used for determining tissue insulin sensitivity with the use of the insulin sensitivity index developed by the present inventors. Thus, the inventions according to the present application make it possible to assess insulin sensitivity and/or determine a predisposition for a disorder connected with insulin resistance in the tested human subject. Determining susceptibility or a predisposition to such a disorder makes it thus possible to undertake early actions aimed at preventing the development of such a disorder and, consequently, also diseases connected therewith.

The methods according to the invention and uses of the insulin sensitivity index according to the invention make it possible to precisely determine tissue insulin sensitivity and/or insulin resistance in a quick, reliable, repeatable and cost-effective way. The results obtained from the method for determining tissue insulin sensitivity according to the invention and the method for identifying insulin resistance and determining a predisposition for a disorder connected with insulin resistance are reliable and repeatable also due to fact that they are independent of insulinaemina fluctuations caused by stress or physical activity. Thus, the methods according to the invention and uses of the insulin sensitivity index according to the invention can be successfully applied to general population, to carry out epidemiological studies, to assess a predisposition for diseases connected with insulin resistance, for early diagnosis of disorders and/or diseases connected with insulin resistance, and for monitoring the progression of such diseases and or for the treatment thereof. In particular, they are suitable for the diagnosis of insulin resistance and for the prognosis, diagnosis and monitoring of a disorder connected with insulin resistance, such as overweight, obesity, impaired glucose tolerance, dyslipidemia, metabolic syndrome, polycystic ovary syndrome, fatty liver, arterial hypertension, atherosclerosis, ischaemic heart disease, cerebral stroke, a neurodegenerative disease, in particular type 2 diabetes.

The inventions as defined in the patent claims are suitable for in general population, in particular in the following human subjects: a person with normal weight, an overweight person, an obese person, a person with increased fasting glycaemia, a person with impaired glucose tolerance, a person with type 2 diabetes, a person with dyslipidaemia, a person with metabolic syndrome, a person with fatty liver, a woman with polycystic ovary syndrome, a person with arterial hypertension, a person with atherosclerosis, a person with ischaemic heart disease, a person after cerebral stroke, a person with a neurodegenerative disease, a woman who had gestational diabetes or gave birth to a baby having over 4 kg body weight, and a first-degree relative of a person suffering from diabetes and/or cardio-vascular diseases. Moreover, the inventions are applicable in human subjects scheduled for bariatric surgery in whom tissue insulin sensitivity or insulin resistance may be a deciding element in the choice of an appropriate bariatric surgery, and human subjects after such surgery in whose case determining tissue insulin sensitivity or insulin resistance may be a deciding element in the choice of appropriate further therapy. The methods according to the invention are not troublesome and inconvenient for the human subject subjected to testing, they minimize his/her pain and the feeling of discomfort connected with a long-lasting testing and multiple insertions of needles required in the methods used so far in the field of the present invention.

The methods according to the invention have been developed by referring the results of determination of a panel of standard laboratory tests and protein concentrations in serum to the measurement of tissue insulin sensitivity by an hyperinsulinemic-euglycemic clamp method, which is the 'gold standard' in the assessment of insulin action *in vivo.*

Definitions

The technical and scientific terms used in the present description have standard meanings used in the field of the present invention. The definitions below have been provided to make it easier for the reader to carry out the present invention.

The term "insulin sensitivity" relates to the ability to properly respond to insulin, which is manifested, first of all, in an increase in skeletal muscle glucose uptake, inhibition of lipolysis and hepatic glucose production. Insulin sensitivity may be measured e.g. by a hyperinsulinemic-euglycemic clamp method (in short, the clamp technique), which is currently the 'gold standard' in determining tissue insulin sensitivity. In this technique, the value of insulin-dependent glucose uptake (M value, hereafter also referred to as variable M) may be expressed in mg/kg of Fat-Free-Mass (FFM)/min.

The term "reduced insulin sensitivity" or "insulin resistance" means decreased response of an organism to endogenous or exogenous insulin as regards the metabolism of carbohydrates, lipids and proteins. In the hyperinsulinemic-euglycemic clamp method, insulin resistance is usually assumed when the value is less than 6 mg/kg FFM/min. In the context of the present invention, insulin resistance is assumed, when the value determined using the insulin sensitivity index defined according to the present invention is 7.5 or less. Furthermore, it is necessary to point out that according to the present invention, a lower result corresponds to lower insulin sensitivity (also within the range below the cut-off point), and a higher result corresponds to higher tissue insulin sensitivity (also within the range above the cut-off point). Thus, the lower the value of the determined insulin sensitivity index, the lower tissue insulin sensitivity (also within the range of the cut-off point), which indicates the presence of insulin resistance in the examined human subject. Identifying insulin resistance makes it possible to diagnose the examined person with a predisposition for a disorder connected with insulin resistance. On the other hand, a higher obtained value of insulin sensitivity index corresponds to higher tissue insulin sensitivity (also within the range above the cut-off point).

Symptoms of insulin resistance are abnormally increased levels of insulin and/or glucose. Insulin resistance predisposes a human subject to the development of a number of abnormalities or disorders, including impaired glucose tolerance, and subsequently type 2 diabetes, atherogenic dyslipidaemia, i.e. hypertriglyceridemia, lowered concentration of high density lipoprotein (HDL) cholesterol in blood and the presence of small, low density lipoprotein (ang. low density lipoportein, LDL) molecules, hyperuricemia, reduced plasma fibrinolytic activity, arterial hypertension, atherosclerosis, cardiovascular diseases (Roriz-Filho, J. et al. Biochim Biophys Acta. 1792: 432-443, 2009).

The term "disorder connected with insulin resistance" relates to a disorder in which the response to insulin action is reduced; in other words, insulin sensitivity is reduced. Disorders connected with insulin resistance, i.e. reduced insulin sensitivity, comprise, among others, overweight, obesity, impaired glucose tolerance, dyslipidemia, fatty liver, metabolic syndrome, polycystic ovary syndrome, cardiovascular diseases, including arterial hypertension, atherosclerosis, ischaemic heart disease, cerebral stroke; neurodegenerative diseases, certain neoplasms, and in particular type 2 diabetes.

The term "sensitivity" in relation to a diagnostic method or test means the ratio of true positives to the sum of true positives and false negatives obtained in a given test. Sensitivity determines the ability of a test to detect human subjects that are diseased or predisposed for the development of a disease. Sensitivity is defined by the following formula:

$$\text{SENSITIVITY} = (\text{PD} / \text{PD} + \text{FU}) \times 100,$$

wherein PD denotes true positives, and FU denotes false negatives.

In the context of the present invention, the term "sensitivity" used in relation to a diagnostic method or test means an index that shows what fraction of all tested subjects with insulin sensitivity lower than 6 mg/kg FFM/min in the test using the clamp technique is correctly identified by a decision criterion based on given cut-off value. The cut-off value is calculated as the ratio of the number of human subjects in whom a method or a test truly indicates insulin resistance truly to all observed human subjects in whom insulin resistance occurred. In the test using the insulin sensitivity index according to the invention, said value is 7.5.

The term "specificity" in relation to a diagnostic method or test means the ratio of true negatives to the sum of true negatives and false positives obtained in a given test. It determines the ability of the test to detect human subjects that are healthy or do not have a predisposition for the development of disease or, in other words, to correctly exclude a disease or a predisposition thereto, and it relates only to the population of healthy human subjects. Specificity is defined by the following formula:

$$\text{SPECIFICITY} = (\text{PU}/\text{PU} + \text{FD}) \times 100\%,$$

wherein PU denotes true negatives, and FD denotes false positives..

In the context of the present invention, the term "specificity" means an index that shows what fraction of human subjects in whom insulin resistance does not occur is correctly identified by a decision criterion based on given cut-off value in a given test. The cut-off value is calculated as the proportion of human subjects with true negative result of the test to all tested human subjects in which insulin resistance was higher than or equal to 6 mg/kg FFM/min in the test using the clamp technique. In the test using the insulin sensitivity index according to the invention, said value is 7.5.

The term "diagnostic power" in relation to a diagnostic method or test means its accuracy. One very common index of diagnostic power is the Area Under Curve (AUC) of Receiver Operating Characteristic (ROC). The value of AUC index falls within the rage from 0 to 1; the higher the value, the better the diagnostic power of a diagnostic method or test. In the test using the insulin insulin sensitivity index according to the invention the value is 0.80.

At present, an accurate assessment of tissue insulin sensitivity is difficult and laborious - it requires the use of at least 2-hour testing by the metabolic clamp technique, with two venipunctures and infusion of insulin and glucose. Considering the fact that it is difficult and laborious, this method is not suitable for use on the population level for routine diagnostic tests in general population. The available indices based on the fasting blood concentration of glucose and insulin are not sufficiently sensitive to diagnose insulin resistance in its early stages, e.g., in young generally healthy human subjects. On the other hand, indices based on glucose and insulin concentrations during an oral glucose tolerance test show low reproducibility (in some test it is only about 30%).

The subject matter of the present invention is a method for determining, *in vitro,* tissue insulin sensitivity in a human subject, consisting in that

a) in a blood sample drawn from a human subject, the following laboratory parameters and concentrations of the following proteins in serum are measured: red blood cells (RBC), alanine aminotransferase (AlAT), C-peptide, sex hormone binding globulin (SHBG), adiponectin, insulin-like growth factor binding protein-1 (IGFBP1);
b) an insulin sensitivity index is determined on the basis of obtained results of measurements specified in a) using the following equation:

Insulin sensitivity index = 11.5847 - RBC x 0.9622 - AlAT x 0.0308 - C-peptide x 1.0718 + SHBG x 0.0239 + Adiponectin x 0.0466 + IGFBP1 x 0.1206,

wherein

RBC is the value of red blood cells count measured in the sample using any method having 4-6 mln/$\mu$l reference interval for general population;
AlAT is the value of alanine aminotransferase activity measured in the sample using any method having 5 - 50 U/l reference interval for general population;
C-peptide is the value of C-peptide concentration measured using any method having 0.003 - 0.04 pmol/ml sensitivity and 0.26 - 2 pmol/ml reference interval for general population;
SHBG is the value of the concentration of sex hormone-binding globulin measured using any method having 0.02 - 0.35 nmol/l sensitivity and 9 - 144 nmol/l reference interval for general population;
Adiponectin is the value of adiponectin concentration measured using any method having 0.15 - 0.5 $\mu$g/ml sensitivity and 6 - 30 $\mu$g/ml reference interval for general population;
IGFBP1 is the value of the concentration of insulin-like growth factor binding protein-1 measured using any method having 0.009 - 0.02 ng/ml sensitivity and 0.2 - 18 ng/ml reference interval for general population.

Calculating the insulin sensitivity index on the basis of the above-described measurements makes it possible to determine a precise and reliable tissue insulin sensitivity. If the obtained value of insulin sensitivity index is 7.5 or less, this indicates reduced tissue insulin sensitivity, i.e. insulin resistance. A lower result corresponds to lower tissue insulin sensitivity (also within the range below the cut-off point), and a higher result corresponds to higher tissue insulin sensitivity (also within the range above the cut-off point). Determining insulin resistance makes it then possible to diagnose a predisposition for the development of a disorder connected with insulin resistance in the tested human subject, as described above.

The subject matter of the invention thus relates also to a method for identifying insulin resistance and determining a predisposition for a disorder connected with insulin resistance in a human subject, consisting in that

a) in a blood sample taken from a human subject, the following laboratory parameters and concentrations of the following proteins in serum are measured: red blood cells (RBC), alanine aminotransferase (AlAT), C-peptide, sex hormone binding globulin (SHBG), adiponectin, insulin-like growth factor binding protein-1 (IGFBP1);
b) an insulin sensitivity index is determined on the basis of obtained results of measurements specified in a) using the following equation:

Insulin sensitivity index = 11.5847 - RBC x 0.9622 - AlAT x 0.0308 - C-peptide x 1.0718 + SHBG x 0.0239 + Adiponectin x 0.0466 + IGFBP1 x 0.1206,

wherein

RBC is the value of red blood cells count measured in the sample using any method having 4-6 mln/$\mu$l reference

interval for general population;

AlAT is the value of alanine aminotransferase activity measured in the sample using any method having 5 - 50 U/l reference interval for general population;

C-peptide is the value of C-peptide concentration measured using any method having 0.003 - 0.04 pmol/ml sensitivity and 0.26 - 2 pmol/ml reference interval for general population;

SHBG is the value of the concentration of sex hormone-binding globulin measured using any method having 0.02 - 0.35 nmol/l sensitivity and 9-144 nmol/l reference interval for general population;

Adiponectin is the value of adiponectin concentration measured using any method having 0.15 - 0.5 $\mu$g/ml sensitivity and 6 - 30 $\mu$g/ml reference interval for general population;

IGFBP1 is the value of the concentration of insulin-like growth factor binding protein-1 measured using any method having 0.009 - 0.02 ng/ml sensitivity and 0.2 - 18 ng/ml reference interval for general population;

c) insulin resistance is identified and a predisposition for a disorder connected with insulin resistance is determined when obtained value of insulin sensitivity index is 7.5 or less. The lower the obtained result of insulin sensitivity index, the lower tissue insulin sensitivity, whereas the higher the result, i.e. over the value of 7.5, means higher tissue insulin sensitivity and it does not indicate insulin resistance or a predisposition for a disorder connected therewith. Obtaining the insulin sensitivity index result of 7.5 allows to identify insulin resistance and indicates a predisposition for the development of a disorder connected with insulin resistance.

The new feature of the present invention consists in a unique combination of the results of laboratory measurements so that they predict tissue insulin sensitivity in a given person in the best way. To carry out the methods according to the invention, only one fasting blood draw is required. In the method for determining insulin sensitivity according to the invention and in the method for identifying insulin resistance and determining a predisposition for a disorder connected with insulin resistance, a specific numerical value is obtained which may be interpreted both as the diagnosis of insulin resistance or the lack thereof (insulin resistance - YES/NO depending on the relation to the cut-off point) and a continuous variable, a higher value of which reflects higher tissue insulin sensitivity.

The methods according to the invention have been developed with the use of complex regression models (ridge, lasso, random forest, supervised principal components regression) as an optimal model of diagnosing insulin resistance and determining a predisposition for the development of a disorder connected therewith. Compared to the known insulin sensitivity tests based, e.g., on a 2-hour oral glucose tolerance test, only one blood draw is required. It should be emphasized that the methods according to the invention are repeatable, specific and sensitive. Moreover, they are characterized by high diagnostic power. The sensitivity of the methods according to the present invention is 90%, the specificity is 68%, whereas the diagnostic power is 0.80.

Insulin resistance can be detected in young human subjects many years before the development of disorders and/or diseases connected therewith. Thus, development of a simple index for assessing the level or degree of tissue insulin sensitivity and insulin resistance finds a significant application in the prevention of type 2 diabetes and other disorders and diseases connected with insulin resistance. The solutions according to the invention are perfectly suitable for this purpose, in particular for the use in general population.

When developing the present invention, 150 young healthy volunteers with different body mass index were subjected to examination and their insulin sensitivity was measured using the metabolic clamp technique which is 'the gold standard' in the assessment of *in vivo* insulin action. Part of the examination involved a detailed clinical characteristics of the examined human subjects and then complex regression models were used in order to detect an array of factors determining in the optimal way tissue insulin sensitivity. This made it possible to develop methods and indices for the uses according to the invention, wherein the results of measurements of selected standard laboratory parameters and the concentrations of selected proteins in serum are used. The method for determining insulin sensitivity according to the invention makes it possible to obtain a specific numerical value and refer it to recommended values. The method allows to assess tissue insulin sensitivity in the examined human subject in a quick and reliable way, on the basis of a blood sample taken from one draw. This makes it possible to carry out the method for identifying insulin resistance and assessing a predisposition for the development of a disorder connected therewith, and thus an accurate early diagnosis of insulin resistance or reduced insulin sensitivity is also possible. It should be emphasized that the methods according to the invention are suitable for use in general population in order to identify insulin resistance and assess a predisposition for diseases connected with insulin resistance or decreased insulin sensitivity, and in particular they can be used in persons with a positive family history of diabetes, in obese persons, persons with arterial hypertension, cardio-vascular disease, neurodegenerative diseases, lipid disorders, in women with polycystic ovary syndrome, in women who had gestational diabetes or gave birth to a baby having over 4 kg body weight and a person before bariatric surgery and/or after such surgery.

The subject matter of the invention relates to a method for assessing tissue insulin sensitivity with the use of selected laboratory parameters and concentrations of selected proteins in serum. The method according to the invention has been developed by referring the results of determination of a panel of standard laboratory tests and concentrations of proteins in serum to the measurement of tissue insulin sensitivity by the hyperinsulinemic-euglycemic clamp technique which is the 'gold standard' in the assessment of insulin action *in vivo.*

More specifically, determining tissue insulin sensitivity and identifying insulin resistance and determining a predisposition for a disorder connected therewith by the methods according to the present invention require only one measurement of RBC count, alanine aminotransferase (AIAt) and the concentration, in serum, of: C-peptide, SHBG (sex hormone binding globulin), adiponectin, IGFBP1 insulin-like growth factor binding protein-1) in a fasting blood sample taken once from the examined human subject. It is also required to use standard, generally known procedures for the preparation of biological material and measurement of selected parameters in whole blood or in serum using commonly known methods and commercially available analyzers. It is possible to use any methods for the measurement of the above laboratory parameters. The results of measurements obtained herein are used for determining insulin sensitivity or insulin resistance with the use of an insulin sensitivity index developed by the present inventors and defined by the following equation:

Insulin sensitivity index = 11.5847 - RBC x 0.9622 - AIAT x 0.0308 - C-peptide x 1.0718 + SHBG x 0.0239 + Adiponectin x 0.0466 + IGFBP1 x 0.1206,

To obtain a value of this index, it is necessary to insert, in the above equation, the values of a single measurement of the following laboratory parameters and protein concentrations in serum:

RBC is the value of red blood cells count measured in the sample using any method having 4-6 mln/$\mu$l reference interval for general population;

AIAT is the value of alanine aminotransferase activity measured in the sample using any method having 5 - 50 U/l reference interval for general population. For the purposes of calculating the insulin sensitivity index according to present invention as described above, the applied value of AIAT activity measurement may exceed the upper limit of the indicated reference range because taking into account such a value and calculating the index according to the invention will not distort the resultant value of the insulin sensitivity index;

C-peptide is the value of C-peptide concentration measured using any method having 0.003 - 0.04 pmol/ml sensitivity and 0.26 - 2 pmol/ml reference interval for general population;

SHBG is the value of the concentration of sex hormone-binding globulin measured using any method having 0.02 - 0.35 nmol/l sensitivity and 9 - 144 nmol/l reference interval for general population;

Adiponectin is the value of adiponectin concentration measured using any method having 0.15 - 0.5 $\mu$g/ml sensitivity and 6 - 30 $\mu$g/ml reference interval for general population;

IGFBP1 is the value of the concentration of insulin-like growth factor binding protein-1 measured using any method having 0.009 - 0.02 ng/ml sensitivity and 0.2 - 18 ng/ml reference interval for general population.

The above-indicated laboratory parameters and concentrations of the specified proteins in serum are measured by standard, commonly known methods applied in clinical diagnostics, with the use of commercially available measurement devices and reagent kits.

For example, the following methods and commercially available kits, analysers and apparatuses can be used to carry out the present invention:

The measurement of red blood cells count may be carried out using any method known and used in the field of clinical analytics; for example, by an impedance method or preferably by a cytometry method with optical laser light scattering analysis. The reference range of red blood cells count for general population is 4 do 6 mln/$\mu$l. For example, a red blood cell count may be determined, among others, using the following analysers: ADVIA 120, ADVIA 360, ADVIA 2120i (Siemens Healthcare Diagnostics, Erlangen, Germany); Sysmex XN-100, Sysmex XN-200, Sysmex XN-3000 (Sysmex, Kobe, Japan); ABX Micros ES 60, ABX Pentra DX-DF-120 (Horiba, Kyoto, Japan).

The measurement of alanine aminotransferase (AIAT) activity in a sample from the examined human subject may be carried out using any method known and used in the field of clinical analytics, within the reference interval of 5 - 50 U/l, for example by a calorimetric method or preferably by a kinetic spectrophotometric method. Human subjects with insulin resistance, in particular with fatty liver, may have elevated AIAT activity; however in such case these values are not very high. If the value exceeds the upper limit of the indicated reference range for AIAT activity, this may mean another disease of the liver. It is possible, however, to incorporate the measured value of this parameter within a range exceeding the upper limit of the reference interval, after excluding other diseases of the liver, e.g., viral hepatitis, or the influence of medicaments. Calculating the insulin sensitivity index according to the invention when AIAT value exceeds the limit of the reference range will not distort the obtained result of the insulin sensitivity index.

For example, the measurement of alanine aminotransferase activity may be carried out using the following commercially available apparatuses: ARCHITECT c4000, ARCHITECT ci4100 (Abbot Diagnostics, Santa Clara, CA, USA); Cobas c111, Cobas Integra 400 Plus (Roche Diagnostics, Burgess Hill, Great Britain); ABX Pentra 400, ABX Pentra C200 (Horiba, Kyoto, Japan).

The measurement of C-peptide concentration may be carried out, for example, by the known enzyme-linked immunosorbent assay (ELISA). One exemplary kit for carrying out such a measurement by ELISA method is the kit of ImmunoAssays S.A. Louvain-La-Neuve, Belgium. The sensitivity of such a kit is 0.01 pmol/ml. The intratest coefficient of variation (CV) (within the kit) is < 8.4%, whereas the intertest coefficient of variation (CV) (between kits) is < 9.2%. Any other kit that allows obtaining analogical measurement sensitivity and coefficients of variation of the measurement can be used. Other exemplary kits are: ELISA kit of DIAGNOSTIC AUTOMATION, Inc., Calabasas, CA, USA, having the sensitivity of 0.025 ng/ml, intratest CV < 9.3% and intertest CV < 9.9%; RIA kit of IBL International GmBH, Hamburg, Germany, having the sensitivity of 0.04 pmol/l, intratest CV and intertest CV < 10.7%; ADVIA Centaur C-peptide test of Siemens Healthcare Diagnostics, Erlangen, Germany, which uses a chemiluminescence method and has the sensitivity of 0.05 ng/ml, intratest CV < 3.3% and intertest CV < 4.1%; Immulite 2000 C-peptide of Siemens Healthcare Diagnostics, Erlangen, Germany, which uses a chemiluminescence method and has the sensitivity of 0.08 ng/ml, the intratest and intratest coefficients of variation < 4.8%; ECLIA kit of Roche Diagnostics, Burgess Hill, Great Britain, which uses a chemiluminescence method and has the sensitivity of 0.003 nmol/l, intratest CV < 4.6% and intertest CV < 5.0%. Conversion of serum C-peptide concentration from ng/ml to pmol/ml: ng/ml / 3 = pmol/ml.

The measurement of SHBG concentration may be carried out, for example, by the known immunoradiometric assay (IRMA) method. One exemplary kit for carrying out such measurement by IRMA method is the kit of ZenTech, Angleur, Belgium. The sensitivity of this kit is 0.26 nmol/l, the intratest coefficient of variation (CV) is < 5.2%, whereas the intertest coefficient of variation (CV) is < 5.8%. Any other kit that allows obtaining analogical measurement sensitivity and coefficients of variation of the measurement can be used. Other exemplary kits are: ELISA kit of Alpco, Salem, NH, USA, having the sensitivity of 0.1 nmol/l, intratest CV < 8.6% and intertest CV < 11.6%; Elecsys SHBG Immunoassay test of Roche Diagnostics, Burgess Hill, Great Britain, which uses a chemiluminescence method and has the sensitivity of 0.35 nmol/l, intratest and intertest CV < 4%.

The concentration of adiponectin may be measured, for example, by the radioimmunoassay (RIA) method or ELISA method. One exemplary kit for carrying out this measurement is commercially available in the company Millipore, MO, USA. The sensitivity of this kit is 0.5 μg/ml. The intratest coefficient of variation (CV) is < 6.2%, whereas the intertest coefficient of variation (CV) is < 9.2%. Any other test or kit that allows obtaining analogical test sensitivity and coefficients of variation of the measurement can be used. Other exemplary kits are: Adiponectin ELISA Kit of Abcam, Cambridge, MA, USA, having the sensitivity of 0.15 μg/ml, intratest CV < 3% and intertest CV < 8.3%; Adiponectin ELISA Kit, Invitrogen ThermoFisher Scientific, Waltham, MA, USA, having the sensitivity of 0.2 μg/ml, intratest CV < 3.8% and intertest CV < 5.5%.

For the measurement of IGFBP-1 concentration, RIA or ELISA method may be used, preferably ELISA method. One exemplary kit for carrying out this measurement is commercially available in the company Mediagnost, Reutlingen, Germany. The sensitivity of such a kit is 0.02 ng/ml. The intratest coefficient of variation (CV) is < 6.8%, whereas the intertest coefficient of variation (CV) is < 7.4%. Any other kit that allows obtaining analogical test sensitivity and coefficients of variation of the measurement may also be used. Other exemplary kits are: Human IGFBP-1 ELISA Kit of LiefSpan Biosciences, Seattle, WA, USA, having the sensitivity of 0.01 ng/ml, intratest CV < 10% and intertest CV < 12%; Human Free IGFBP-1 Quantikine ELISA kit of R & D Systems, Minneapolis, MN, USA, having the sensitivity of 0.014 ng/ml, intratest CV < 5.6% and intertest CV < 9.5%.

To calculate the present insulin sensitivity index it is necessary to take into account the values of parameters obtained in the respective units of measure indicated above: RBC count expressed in mln/μl, AIAT activity - in U/l, C-peptide concentration - in pmol/ml, SHBG concentration - in nmol/l, adiponectin concentration - in μg/ml, IGFBP1 concentration - in ng/ml. If the value of a parameter used to calculate the index is expressed in other units, is should be converted into units given above.

If the measurement methods used to determine the parameters necessary to calculate the insulin sensitivity index according to the present invention have different sensitivity and/or a different reference interval compared to the above-indicated recommended measurement methods, it is possible to correct the calculations, i.e. if the mean value of given parameter significantly differs from the reference interval, the multiplier should be corrected accordingly, i.e. if the mean value of the range is five times higher, the multiplier of the given parameter should be reduced five times.

Interpretation of results

The value of 7.5 or less obtained using the above-described insulin sensitivity index indicates reduced tissue insulin sensitivity, i.e. insulin resistance. A lower result corresponds to reduced, lower tissue insulin sensitivity, also within the range below the cut-off point, whereas a higher result obtained, corresponds to higher tissue insulin sensitivity, also within the range above the cut-off point. Thus, it is also possible to determine a predisposition for a disorder connected with insulin resistance or diagnose the lack of a predisposition for such a disorder.

The sensitivity of the methods according to the present invention is 90%, and the specificity is 68%. The area under the receiver operating characteristic (ROC) curve, i.e. the diagnostic power, is 0.80.

Experimental verification

The method according to the invention was tested in a statistical analysis in the so-called learning sample and testing sample. Then, it was verified in samples taken from earlier studies done by the present inventors, which encompassed 400 human subjects in whom insulin sensitivity was measured by the metabolic clamp method. The index was successfully verified as a diagnostic biomarker, more specifically as a biomarker of reduced tissue insulin sensitivity, i.e. insulin resistance, in groups of human subjects with normal body weight, obesity and overweight, in human subjects with impaired glucose tolerance, and in women with polycystic ovary syndrome.

The study was carried out in a group of 150 young healthy volunteers with different body mass index in whom insulin sensitivity was measured using the metabolic clamp technique which represents 'the gold standard' in the assessment of insulin action *in vivo.* Part of the study involved detailed clinical characteristics of the examined human subjects, the assessment of basis laboratory parameters and the measurement of the serum concentration of a panel of proteins.

Then, in order to detect an array of factors which determines tissue insulin sensitivity in the optimal way, complex regression models were constructed: the linear model with regularization (ridge and lasso regression), the supervised principal components model, and the random forest model. The randomly selected learning sample and testing sample were used in the analyses. The result of the clamp test, determining tissue insulin sensitivity, was subjected to categorization with the value of 6 mg/kg FFM/min adopted as the cut-off point.

The inventors resigned from the division of the quality variable (tissue insulin sensitivity) according to the median and tertiles in the examined population due to the relationship of these values with the examined sample. Regression models were constructed for all examined variables and for the individual areas of variables (clinical characteristics, biochemical characteristics, gene expression in whole blood, gene expression in tissues, protein concentrations in blood). It was observed that the areas of variables concerning the clinical characteristics and gene expression make it possible to predict insulin sensitivity independently of each other. Additionally, the whole area of the clinical characteristics, comprising also measurements obtained during the oral glucose tolerance test, as well as the same area limited to the results obtained in fasting tests only were analysed.

The sensitivity and specificity of the given test were taken into account. Sensitivity is an index that indicates what fraction of all the examined (human) subjects having less than 6 insulin sensitivity value is identified by the decision criterion based on the given cut-off value. It is calculated as the proportion of the number of human subjects in whom the test truly indicates insulin resistance to all observed human subjects in whom it occurred. Specificity is an index that indicates what fraction on patients in whom insulin resistance does not occur is correctly identified by the decision criterion based on given cut-off value. It is calculated as the proportion of the number of human subjects with truly negative result of the test to all observed human subjects in whom insulin sensitivity was greater than or equal to 6.

Statistical analysis

The statistical analysis was carried out using STATISTICA 15.5 software (StatSoft Poland, Cracow). Before carrying out statistical tests, the assumptions of normality of distribution and homogeneity of variance in the compared groups of volunteers were verified. Normality of distribution was assessed using the Shapiro-Wilk test, whereas homogeneity of variance was assessed using the Levene test and the Brown-Forsythe test. Then, tests for the significance of differences between the groups of volunteers were carried out. For the comparison of 2 groups, the non-parametric Mann-Whitney test was used, and the parametric test t-Student - for the independent samples. To analyse the distribution of quality variables in groups determined by variable M (insulin sensitivity expressed in mg/kg FFM/min), Pearson's chi-square test of independence was carried out. Correlation and simple regression analysis were carried out for the independent variables and between the independent variables and the dependent variable M using the Spearman's rank correlation coefficient analysis and Pearson's linear correlation r.

Due to the large number of variables and the fact that said number was greater than the number of observations, special techniques of data analysis were used, as described below. To detect an array of factors determining tissue insulin sensitivity in the optimal way, complex regression models were constructed: a linear model with regularization (ridge and lasso regression), a supervised principal components model and a random forest model. A randomly selected learning sample and a test sample were used in the analyses. The learning sample was used for the construction of models and the testing sample was used for the assessment thereof. The division of the set into two samples is consistent with the good practice applied in the construction of models and was aimed at increasing the reliability of the quality assessment of the prepared models. The size of the learning sample was established at 80% of the size of the whole sample. Thus, 120 volunteers were assigned to the learning sample and 30 volunteers - to the test sample. Human subject were assigned to the samples in a random manner taking into account the goodness of fit of variable M in both groups. The result of the clamp test, determining tissue insulin sensitivity, was subjected to categorization with the value of 6 mg/kg FFM/min adopted as the cut-off value. The inventors resigned from the division of variable M according to the median and tertiles in the examined population due to the relationship of these values with the examined sample. The value of 6 mg/kg FFM/min was adopted on the basis of earlier observations by the present inventors and on the basis of literature. Such cut-off point results in that 60 human subjects (40% of the examined group) were assigned to the group with reduced insulin sensitivity.

Regression models were constructed for all examined variables and for individual areas of variables (clinical characteristics, biochemical characteristics, gene expression in complete blood, gene expression in tissues, protein concentrations in blood). It was observed that the areas of variables concerning clinical characteristics, biochemical characteristics and gene expression make it possible to predict insulin sensitivity independently of each other. Models were constructed using STATISTICA Data Miner (StatSoft, 2011]) software and R package (packages: impute (Hastie T, Tibshirani T, Narasimhan B, Chu G. impute: impute: Imputation for microarray data. 2014, R package version 1.32.0), glmnet (Friedman J, Hastie T, Tibshirani R. (2010). Regularization Paths for Generalized Linear Models via Coordinate Descent. Journal of Statistical Software, 2010, 33: 1-22. URL http://www.jstatsoft.org/v33/i01) and superpc (Bair E., Tibshirani R. superpc: Supervised principal components. 2012, R package version 1.09. http://CRAN.R-project.org/package=superpc).

The models described can be divided into two groups - models whose purpose is to best predict the value of variable M on the basis of all variables and models that are easier to be interpreted due to reduced number of predicators. The quality of goodness of fit of the models from the first group (ridge and random forest regression) is slightly better compared to the remaining ones. On the other hand, the lasso model and the 'supervised principal components' model reduce the number of variables and make it possible to find significant diagnostic factors and thus help to understand the studied phenomenon. In particular, the 'supervised principal components' model, apart from indicating essential factors that have an influence on the value of variable M, shows relations between predicators. To develop the insulin sensitivity index, the 'supervised principal components' model was finally chosen, which predicted variable M in an optimal way with simultaneously reduced number of predicators (unlike in the ridge regression or random forest model which use all examined variables).

In all analyses presented in the working examples of the present invention the adopted statistical significance level was $p < 0.05$.

The applied regression models made it possible to develop an insulin sensitivity index determining tissue insulin sensitivity in the optimal way, also as compared with the above-mentioned indices used before. The index developed in accordance with the invention is independent of insulinaemia and glucose fluctuations and makes it possible to obtain the determination with 90% sensitivity and 68% specificity. The diagnostics power is 0.80 and it is greater than the diagnostic power of previously known indices. Thanks to that, the insulin sensitivity index developed by the present inventors may be used as a diagnostics biomarker, as a biomarker of insulin resistance, and as a prognostic biomarker of a disorder connected with insulin resistance, as well as a biomarker for determining a predisposition for a disorder connected with insulin resistance.

It should be added that the developed method for determining tissue insulin sensitivity and the method for identifying insulin resistance and determining a predisposition for a disorder connected therewith, and the insulin sensitivity index according to the invention were analysed in relation to the tissue insulin sensitivity measured by the clamp technique as a continuous variable, i.e. without categorising human subjects with high and low tissue insulin sensitivity, that is, by the correlation analysis method. In that case the index also proved to be better compared to previously known indices. It should be noted that the examined group consisted of young, generally healthy human subjects, in such circumstances correlations of different indices with the value obtained in the clamp technique are usually weaker than in case of a test performed on groups having a significant level of insulin resistance. In this context, it is worth mentioning that the methods for determining tissue insulin sensitivity and identifying insulin resistance and determining a predisposition to a disorder connected with insulin resistance, as developed according to the invention, as well as the insulin sensitivity index according to the invention showed a strong, significant correlation with the value of insulin sensitivity obtained in the clamp test ($r = 0.57$, $p<0.0001$), which was stronger than HOMA-IR ($r = -0.34$, $p < 0.001$) and QUICKI ($r = 0.34$, $p<0.001$). The methods for determining tissue insulin sensitivity and insulin resistance, as developed according to the invention, and the insulin sensitivity index according to the invention also proved to be better in relation to the Matsuda index (the correlation Matsuda vs the value from the clamp technique: $r = 0.38$, $p < 0.001$), which is even more important considering that the Matsuda index requires the measurement of glucose and insulin concentrations in blood during a 2-hour glucose tolerance test, whereas the developed method for determining tissue insulin sensitivity and the insulin sensitivity index according to the invention requires only a fasting blood sample from one blood draw.

To sum up, in accordance with the present invention a method for determining tissue insulin sensitivity and a method for identifying insulin resistance and determining a predisposition for a disorder connected with insulin resistance based on *in vitro* measurements of standard laboratory parameters and concentrations of specific proteins in serum have been developed. The methods according to the invention make it possible to determine tissue insulin sensitivity of the examined human subject and to obtain a specific numerical value determining tissue insulin sensitivity, and by relating it to recommended values, to determine whether insulin sensitivity is reduced or not. This enables a precise identification of insulin resistance, early diagnosis of insulin resistance and the assessment of a predisposition for a disorder connected with insulin resistance, on the basis of results obtained from a single fasting blood draw.

The above-described insulin sensitivity index according to the invention may be used as a biomarker applied in the methods for determining a predisposition for disorders connected with insulin resistance, such as type 2 diabetes, first of all, as well as cardiovascular diseases, including arterial hypertension, atherosclerosis, ischaemic heart disease, cerebral stroke; overweight, obesity, impaired glucose tolerance, dyslipidaemia, metabolic syndrome, fatty liver, neurodegenerative diseases, in endocrine syndromes, including syndromes associated with impaired fertility, for example in polycystic ovary syndrome.

The above-described insulin sensitivity index according to the invention is capable of routine application in clinical practice, for tests in general population, in human subjects with normal body weight, for the assessment of a predisposition for diseases connected with insulin resistance, in particular in human subjects: with a positive family history of diabetes, with increased fasting glicaemia, with impaired glucose tolerance, overweight, obesity, with lipid disorders, with dyslipidaemia, with metabolic syndrome, with fatty liver, with cardiovascular diseases, including hypertension, with atherosclerosis, with ischaemic heart disease, after cerebral stroke, with neurodegenerative diseases, in women who had gestational diabetes or gave birth to a baby with over 4 kg body weight, or in first-degree relatives of a person suffering from diabetes and/or cardiovascular diseases, or persons before bariatric surgery and/or after such surgery.

The present invention will be illustrated below by examples and figures which, however, are not meant to limit, in any way, the scope of protection of the invention as defined in the patent claims.

**Brief Description of Drawings**

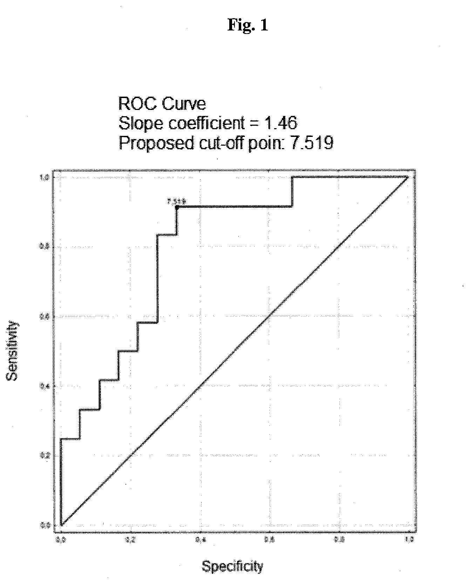

Figure 1 shows a curve for the testing sample. For the cut-off point of 7.519 the sensitivity of the insulin sensitivity index according to the invention is 90%, whereas the specificity of the insulin sensitivity index according to the invention is 68%.

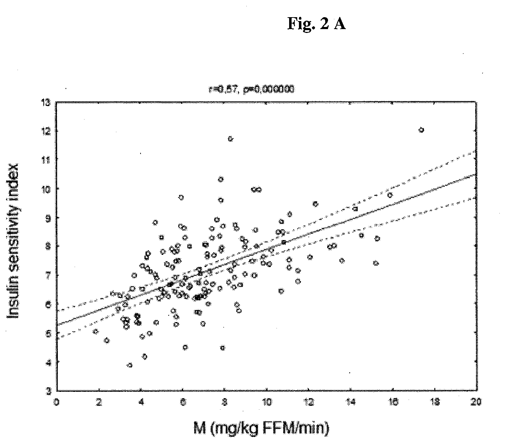

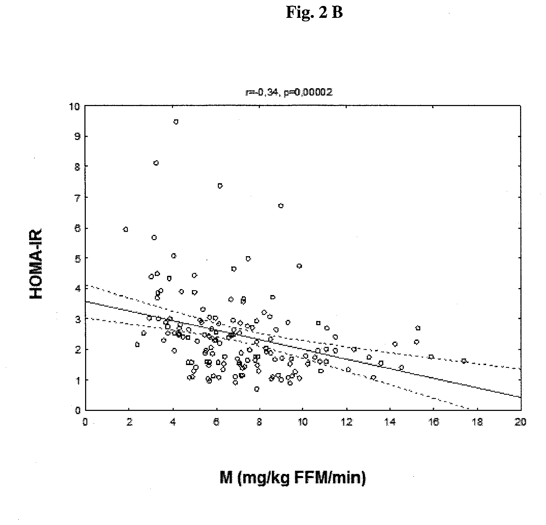

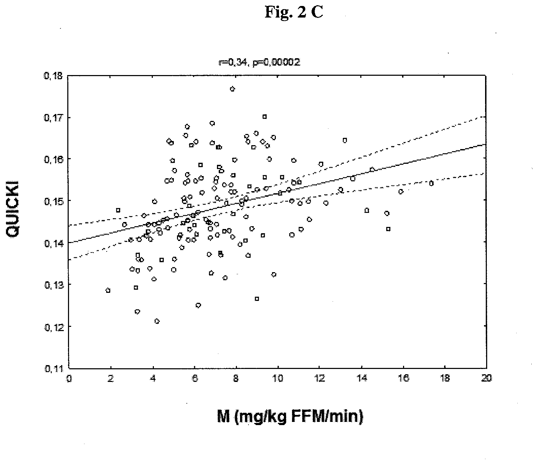

Figure 2 shows the results of the analysis of the correlation between the insulin sensitivity index according to the invention and the results of insulin sensitivity obtained in the clamp test and with the use of HOMA-IR and QUICKI indices. In Figure 2A, the statistically significant correlation with the value of variable M ($r = 0.57$, $p < 0.000001$) can be seen. Figure 2B shows the obtained statistically essential correlation which is stronger than that using HOMA-IR index ($r = -0.34$, $p = 0.00002$), whereas Figure 2C shows the statistically significant correlation with the QUICKI index ($r = 0.34$, $p = 0.00002$).

**Examples**

*Examined group*

150 generally healthy human subjects aged 18 - 35: 117 men and 33 women were enrolled to the study. Volunteers for the study were recruited through local advertisements and among medical personnel and students. All examined human subjects were non-smokers, without concomitant diseases (ischaemic heart disease, hypertension, infections, allergies, diabetes, neoplasms, diseases of the kidneys, the liver and the endocrine system), without sings of malnutrition and without severe obesity (BMI - body mass index between 18.9 and 40 kg/m$^2$), and were not taking any medicaments. Their body weight was stable in the previous 3 months. None of the women had any menstrual disorders, all of them were examined between the 3$^{rd}$ and 5$^{th}$ day of the menstrual cycle.

The inventors received an approval of the Bioethics Committee of the Medical University in Bialystok to carry out the examination. All the examined human subjects gave a written consent for their participation in the examination after reading information concerning the examination and explaining any doubts with a practitioner participating in the project.

*Study protocol and methods*

Stage I

*Medical examination and anthropometric measurements*

The study was carried out in two stages. All tests were carried out under fasting conditions, after at least 10-hour period without meal. In the first stage, full medical examination was performed and anthropometric measurements were made in subsequently examined volunteers. The following measurements were taken: body weight, height, waist circumference (the smallest circumference between the costal arch and the iliac crest), hip circumference (the widest place between the waist and thighs), body fat percentage by bioelectrical impedance analysis method using Tanita TBF-511 Body FAT Analyzer (Tanita Copr., Tokio, Japan). Then, BMI was calculated as body mass x height $^{-2}$ and expressed in $kg/m^2$, Waist-to-Hip Ratio (WHR), body Fat Mass (FM) and body Fat-Free Mass (FFM) on the basis of body weight and fat percentage in the organism. In further tests were enrolled human subjects who were non-smokers, had no concomitant diseases, who had no deviations in the basic examination and who were not taking any medicaments.

*Glucose tolerance assessment and preliminary biochemical examination*

After the initial medical qualification, a standard oral glucose tolerance test (OGTT) was performed. During the test the blood was drawn from the basilic vein in fasting conditions, before the administration of glucose, and in 30th, 60th and 120th minute after oral 75g-glucose load in order to determine plasma glucose concentrations as well as insulin and free fatty acids concentrations in serum. Plasma glucose concentrations were directly determined during the examination. Additionally, the following standard laboratory parameters were directly determined in the fasting blood with the use of standard reagent kits and diagnostic apparatuses: complete blood count with differential, coagulation, lipids, electrolytes, uric acid, creatinine clearance, bilirubin, total protein, and proteinogram, the activity of: aspartate aminotransferase (AspAt), alanine aminotransferase (AlAT), alkaline phosphatase, gamma-glutamyl transpeptidase (GGTP); iron concentration, ferritin concentration, total iron binding capacity (TIBC), C-reactive protein (hsCRP), TSH, fT3 and fT4, cortisol and sex hormones. Urinalysis and microalbuminuria test were also performed in a routine way. The samples of serum and plasma were stored at -80°C until the performance of the remaining analyses. Human subjects who were enrolled to the second stage of the study had normal glucose tolerance and normal results of the laboratory tests panel.

Stage II

In stage II, a biopsy of the vastus lateralis muscle and the subcutaneous adipose tissue was performed and then tissue insulin sensitivity was measured using the hyperinsulinemic-euglycemic clamp technique in the known way as described, e.g., in publications: Karczewska-Kupczewska M, Lelental N, Adamska A, Nikolajuk A, Kowalska I, Górska M, Zimmermann R, Kornhuber J, Strączkowski M, Lewczuk P. The influence of insulin infusion on the metabolism of amyloid β peptides in plasma. Alzheimers Dement 2013, 9: 400-405; and Karczewska-Kupczewska M, Kowalska I, Nikolajuk A, Adamska A, Zielińska M, Kamińska N, Otziomek E, Górska M, Strączkowski M. Circulating brain-derived neurotrophic factor concentration is downregulated by intralipid/heparin infusion or high-fat meal in young healthy male subjects. Diabetes Care 2012, 35: 358-362. The measurements were performed at least 5 days after OGTT, in the morning, in fasting conditions.

*Biopsy of vastus lateralis muscle and subcutaneous adipose tissue*

Prior to the clamp test, a biopsy of the vastus lateralis muscle and the subcutaneous adipose tissue from the umbilical region was performed under local anaesthetic: 1% lidocaine, after a small incision of the skin (about 1 cm). The biopsy of the muscle was performer using a special needle (Popper and Sons, New Hyde Park, NY, USA), about 15 cm above the patella (knee cup). The biopsy of the adipose tissue was performed using a Byron Accelerator III needle. The material from the biopsy was placed in -80°C in order to preserve it until mRNA and protein were isolated. Gene expression in the tissues was measured by a mRNA microarray technique, and then Real Time PCR.

*Example 1*

*Measurement of tissue insulin sensitivity and identification of high and low tissue insulin sensitivity*

In order to perform the test using the clamp technique, 2 IV needles were inserted into the volunteers' 2 basilic veins. One of the needles was intended for simultaneous infusion of glucose and insulin, whereas the other one, placed on the opposite side, was used for drawing blood in order to determine current glycaemia. The limb from which the blood was drawn was heated by means of an electric cushion up to about 60° C in order to arterialize venous blood. During the test, which lasted 2 hours, intravenous infusion of short-acting insulin (Actrapid HM, Novo Nordisk, Copenhagen, Denmark) was used: in the first 5 min - at the rate of 80 mU x $m^{-2}$ x $min^{-1}$, from 5th minute - 60 mU x $m^{-2}$ x $min^{-1}$, and then, from 10th min. continuous infusion at the rate of 40 mU x $m^{-2}$ x $min^{-1}$, to obtain stable hyperinsulinemia, sufficient to inhibit endogenous insulin secretion, to suppress lipolysis and reduce endogenous glucose production to 10-15% of the baseline values. Glucose concentration during the clamp test was determined every 5 minutes using YSI 2300 Stat Plus analyser. Minimal amount of blood was needed to determine glycaemia and the result was obtained after 15-45 sec. During the clamp test the analyser was automatically calibrated - at least every 30 min. In 4th minute of the test, the 20% glucose infusion (1.11 mol/l) began, initially at the rate of 2 mg x kg$^{-1}$ x min$^{-1}$, then from 10th minute, glucose infusion was controlled manually depending on the current glucose concentrations, so that glycaemia was closest to 90 mg/dl (5.0 mmol/l). In all human subjects, glucose flow rate was controlled empirically.

When using the above-described protocols, stable metabolic conditions are obtained - stable hyperinsulinemia. In the second hour of the test stable glucose concentrations and stable glucose flow rate are obtained. In such situation, the glucose flow rate equals the rate of glucose metabolism in tissues, primarily in the skeletal muscles. The adopted value of the insulin-dependent glucose uptake in the organism is the average glucose infusion rate in the last 40 minutes of the clamp test, corrected taking into account slight changes in glycaemia in this time interval and converted into FFM (value M).

Moreover, blood was taken prior to the clamp test in order to measure concentrations of selected proteins in serum, to perform proteomic analysis and isolation of peripheral blood mononuclear cells (PBMC) for the analyses of gene expression using the mRNA microarray technique and Real Time PCR.

*Analytical methods*

Plasma glucose concentration was measured using an enzymatic method with a biochemical analyser (YSI 2300 STAT PLUS). RBC count was determined by a cytometry method with optical laser light scattering analysis with the use of ADVIA2120I analyser. Lipids concentration in serum was determined by a calorimetric method with the use of Cobas c111 analyser (Roche Diagnostics, Mannheim, Germany). The activity of: AspAt, AlAt was determined by a kinetic spectrophotometric method with the use of ARCHITECTc4000 analyser (the total coefficient of variation - CV for AlAt was below 5.3%). Concentration of hsCRP was measured using a nephelometric method (Dade Behring, Marburg, Germany).

Serum insulin concentration was measured using an immunoradiometric assay method (RMA; DIAsource ImmunoAssays S.A., Nivelles, Belgium) having 1 mIU/ml sensitivity and intratest and intertest values of CV which were: below 2.2% and 6.5%, respectively. Serum FFA concentration was measured using a commercially available kit (Wako Chemicals, Richmond, VA, USA).

The following fasting concentrations were also measured prior to the clamp test: C-peptide, proinsulin, sex hormone-binding globulin(SHBG), mannan-binding lectin (MBL)), tumor necrosis factor $\alpha$ (TNF$\alpha$) and soluble forms of its receptors (sTNFR1 and sTNFR2), interleukin 6 (IL-6), soluble interleukin receptor subunits (sIL-6R and sgp130), interleukin 9, 10, 12, 18 (IL-8, IL-10, IL-12, IL-18, respectively), IL-12p40, adiponectin, leptin, visfatin, resistin, retinol-binding protein 4 (RBP4), ghrelin, E-slectin, soluble intercellular adhesion molecule 1 (sICAM1), soluble vascular cell adhesion molecule (sVCAM), monocyte chemoattractant protein 1 (MCP1), endothelin 1, brain-derived neurotrophic factor (BDNF), brain natriuretic peptide (BNP), fibroblast growth factor 21 (FGF21), insulin-like growth factor I (IGF-I), IGF-I-binding proteins (IGFBP-1 and IGFBP-3), a matrix metalloproteinase 9 (MMP9), macrophage migration inhibitory factor (MIF), macrophage inhibitory cytokine 1 (MIC-1), sclerostin, osteoprotegerin, osteocalcin, receptor activator for nuclear factor $\kappa$ B ligand (RANKL). Serum adiponectin concentration was measured using a radioimmunoassay (RIA) method, whereas SHBG - an immunoradiometric assay (IRMA) method. The concentrations of all the remaining above-mentioned factors in serum were measured by an enzyme-linked immunosorbent assay (ELISA). The islet cell antibodies titre was also determined.

All parameters were determined using commercially available kits and analysers in accordance with producers' recommendations.

*Statistical analysis*

The statistical analysis was carried out using STATISTICA 15.5 software (StatSoft Poland, Cracow). Before carrying out statistical tests, the assumptions of normality of distribution and homogeneity of variance in the compared groups of volunteers were verified. Normality of distribution was assessed using the Shapiro-Wilk test, whereas homogeneity of variance was assessed using the Levene test and the Brown-Forsythe test. Then, tests for the significance of differences between the groups of volunteers were carried out. For the comparison of 2 groups, the non-parametric Mann-Whitney test was used, and the parametric test t-Student - for the independent samples. To analyse the distribution of quality variables in groups determined by variable M, Pearson's chi-square test of independence was carried out. Correlation and simple regression analysis were carried out for the independent variables and between the independent variables and the dependent variable MA using the Spearman's rank correlation coefficient analysis and Pearson's linear correlation r.

In order to detect an array of factors which determine tissue insulin sensitivity in the optimal way, complex regression models were constructed: a linear model with regularization (ridge and lasso regression), a supervised principal components model, and a random forest model. A randomly selected learning sample and a testing sample were used in the analyses. The size of the learning sample was established at 80% of the size of the whole sample. Thus, 120 volunteers were assigned to the learning sample and 30 volunteers - to the testing sample. Human subject were assigned to the samples in a random manner taking into account the goodness of fit of the distribution of dependent variable M in both groups. The result of the clamp test determining tissue insulin sensitivity was subjected to categorization with the value of 6 mg/kg FFM/min adopted as the cut-off point. The inventors resigned from the division of variable M according to the median and tertiles in the examined population due to the relationship of these values with the examined sample. The value of 6 mg/kg FFM/min was adopted on the basis of earlier observations by the present inventors and on the basis of literature. Such cut-off point results in that 60 human subjects (40% of the examined group) were assigned to the group with reduced insulin sensitivity.

Regression models were constructed for all examined variables and for individual areas of variables (clinical characteristics, biochemical characteristics, gene expression in whole blood, gene expression in tissues, protein concentrations in blood). It was observed that the areas of variables concerning the clinical characteristics and gene expression make it possible to predict insulin sensitivity independently of each other. The models were constructed using STATISTICA Data Miner software (StatSoft, 2011]) and R package (packages: impute, glmnet and superpc).

In all analyses the adopted statistical significance level was $p < 0.05$.

*Results*

Clinical characteristics of human subjects with high and low tissue insulin sensitivity determined according to the clamp test are presented in Table 1.

Human subjects with low tissue insulin sensitivity were characterized by higher systolic pressure (p = 0.046), body weight (p = 0.002), BMI (p = 0.013), waist circumference (p = 0.0004), hip circumference (p = 0.001) and Waist-to-Hip Ratio (WHR, p = 0.039), plasma glucose concentration (p = 0.023), serum insulin concentration (p = 0.0027), higher concentrations of total cholesterol (p = 0.007), triglycerides (p = 0.0001) and LDL cholesterol (p = 0.037), and lower HDL cholesterol concentration (p = 0.03), higher red blood cells count (RBC, p=0.0018), hemoglobin concentration in blood (Hb, p = 0.008), haematocrit (Ht, p = 0.019), white blood cells count (WBC, p = 0.003), AlAT activity (p = 0.002), concentration of sodium (p=0.001), uric acid (p = 0.004), creatinine clearance (p = 0.003), higher TIBC (p = 0.02), ferritin (p=0.02) and E selectin (p = 0.0018) (Table 1). The group of human subjects with low tissue insulin sensitivity were also found to have a higher serum C-peptide concentration (p = 0.0007), lower concentration of SHBG (p = 0.0016), adiponectin (p = 0.0017) and IGFBP-1 (p = 0.019) (Table 1 below). In accordance with the assumptions, the examined groups differed in the tissue insulin sensitivity measured during the hyperinsulinemic-euglycemic clamp test (Table 1 below). In all examined human subjects a negative titre of anti-islet cell antibodies was reported.

Table 1. Clinical characteristics of human subjects with high (M > 6 mg/kg FFM/min; n = 90) and low (M < 6 mg/kg FFM/min; n = 60) tissue insulin sensitivity.

| Parameter | High insulin sensitivity | Low insulin sensitivity |
|---|---|---|
| Age (years) | 23.41±2.76 | 23.37±3.18 |
| Systolic pressure (mmHg) | 121.53±14.63 | 125.80±9.21* |
| Diastolic pressure (mmHg) | 76.38±8.22 | 78.32±7.69 |
| Body weight (kg) | 76.67±13.87 | 84.48±17.03* |
| BMI (kg/m$^2$) | 24.47±3.67 | 26,14±4.44* |
| Waist circumference (cm) | 84.91±9.70 | 91.65±12.97* |
| Hip circumference (cm) | 98.29±8.90 | 103.10±8.50* |
| WHR | 0.86±0.06 | 0.89±0.08* |
| % fat | 21.72±8.86 | 22.85±9.06 |
| Plasma glucose (mg/dl) | 85.53±7.92 | 88.67±8.64* |
| Serum insulin ($\mu$IU/ml) | 10.10±5.22 | 13.01±6.42* |
| Serum FFA (mmol/l) | 0.65±0.26 | 0.72±0.28 |
| Cholesterol (mg/dl) | 164.77±29.01 | 178.84±32.38* |
| Triglycerides (mg/dl) | 76.48±32.40 | 104.65±54.67* |
| HDL cholesterol (mg/dl) | 61.31±12.34 | 56.98±10.73* |
| LDL cholesterol (mg/dl) | 95.83±28.66 | 107.26±36.71* |
| RBC (mln/$\mu$l) | 4.96±0.42 | 5.17±0.34* |
| Hb (g/dl) | 14.64±1.21 | 15.13±0.90* |
| Ht (%) | 43.32±3.42 | 44.53±2.46* |
| WBC (thousand/$\mu$l) | 5.90±1.11 | 6.50±1.33* |

(continued)

| Parameter | High insulin sensitivity | Low insulin sensitivity |
|---|---|---|
| Blood pellets (thousand/μl) | 240.18±41.63 | 244.17±48.62 |
| Fibrinogen (mg/dl) | 281.82±49.60 | 287.22±51.07 |
| AspAt (U/l) | 23.70±9,56 | 23.92±7.47 |
| A1AT (U/l) | 21.98±15.64 | 28.58±13.12* |
| Sodium (mmol/l) | 138.53±2.36 | 139.87±2.57* |
| Potassium (mmol/l) | 4.36±0.31 | 4.42±0.35 |
| Total calcium (mmol/l) | 2.43±0.12 | 2.46±0.11 |
| Ferrum (μg/dl) | 106.41±44.37 | 94.02±41.48 |
| TEBC (μg/dl) | 304.67±93.65 | 338.84±74.28* |
| Ferritin (ng/ml) | 60.28±47.16 | 78.66±47.99* |
| Uric acid (mg/dl) | 4.80±1.12 | 5.34±.13* |
| Creatinine (mg/dl) | 0.92±0.19 | 0.93±0.11 |
| Creatinine clearance (ml/min) | 131.82±26.40 | 146.16±31.11* |
| Total protein (g/dl) | 7.37±0.39 | 7.47±0.35 |
| Albumins (%) | 61.21±3.66 | 62.23±4.04 |
| β-globulins (%) | 11.68±1.20 | 12.09±1.46 |
| γ-globulins (%) | 15.61±8.02 | 13.85±2.49 |
| hsCRP (mg/l) | 0.61±0.56 | 0.81±0.77 |
| C-peptide (pmol/ml) | 0.59±0.22 | 0.74±0.31* |
| Proinsulin (pmol/l) | 0.96±0.98 | 1.55±0.63 |
| SHBG (nmol/l) | 31.74±16.84 | 23.67±11.50* |
| Adiponectin (μg/ml) | 17.53±7.32 | 13.95±5.56* |
| IGF-I (ng/ml) | 283.53±60.37 | 285.69±66.14 |
| IGFBP-1 (ng/ml) | 4.04±3.16 | 2.83±2.89* |
| IGFBP-3 (ng/ml) | 2595.83±466.77 | 2687.57±535.34 |
| Selectin E (ng/ml) | 33.70±14,36 | 41.37±14.77* |
| M (mg/kg FFM/min) | 8.94±2.51 | 4.57±1.04* |

\* p < 0.05 vs the group with high insulin sensitivity

The most significant results of one-way analyses of correlations between the examined parameters and the dependent variable M are presented in Table 2 in the form of a ranked variables according to the correlation coefficient. As can be seen in Table 2 below, the following parameters showed the strongest correlation with M: SHBG, waist circumference, IGFBP-1, RBC, C-peptide, AIAT, triglycerides, GGTP, body weight, insulin concentration in serum, serum adiponectin concentration, HDL cholesterol.

Table 2. Rank of correlations of the most essential parameters examined in fasting conditions with the dependent variable M

| Parameter | Spearman's R | p | Pearson's R | p |
|---|---|---|---|---|
| SHBG | 0.421 | 0.000 | 0.411 | 0.000 |
| Waist | -0.384 | 0.000 | -0.389 | 0.000 |
| IGFBP-1 | 0.455 | 0.000 | 0.388 | 0.000 |
| RBC | -0.368 | 0.000 | -0.364 | 0.000 |
| C-peptide | -0.422 | 0.000 | -0.357 | 0.000 |
| AIAt | -0.374 | 0.000 | -0.342 | 0.000 |
| Triglycerides | -0.334 | 0.000 | -0.329 | 0.000 |
| GGTP | -0.320 | 0.000 | -0.327 | 0.000 |
| Body weight | -0.314 | 0.000 | -0.322 | 0.000 |
| Insulin | -0.394 | 0.000 | -0.319 | 0.000 |
| Adiponectin | 0.313 | 0.000 | 0.305 | 0.000 |

(continued)

| Parameter | Spearman's R | p | Pearson's R | p |
|---|---|---|---|---|
| HDL cholesterol | 0.325 | 0.000 | 0.306 | 0.000 |
| Sex - female | 0.283 | 0.000 | 0.298 | 0.000 |
| Hb | -0.318 | 0.000 | -0.291 | 0.000 |
| WBC | -0.276 | 0.001 | -0.286 | 0.000 |
| Ht | -0.306 | 0.000 | -0.279 | 0.001 |
| BMI | -0.270 | 0.001 | -0.278 | 0.001 |
| Systolic pressure | -0.256 | 0.002 | -0.270 | 0.001 |
| Sodium | -0.310 | 0.000 | -0.264 | 0.001 |
| Ferritin | -0.311 | 0.000 | -0.264 | 0.001 |
| Cholesterol | -0.268 | 0.001 | -0.262 | 0.001 |
| Creatinine clearance | -0.215 | 0.009 | -0.260 | 0.001 |
| Creatinine | -0.243 | 0.003 | -0.250 | 0.002 |
| Uric acid | -0.297 | 0.000 | -0.249 | 0.002 |
| Proinsulin | -0.278 | 0.001 | -0.243 | 0.003 |
| Selectin E | -0.309 | 0.000 | -0.242 | 0.003 |
| $\beta$-globulins | -0.225 | 0.006 | -0.242 | 0.003 |
| MCV | 0.241 | 0.003 | 0.222 | 0.006 |
| Total calcium | -0.192 | 0.019 | -0.223 | 0.006 |
| TIBC | -0.203 | 0.015 | -0.225 | 0.006 |
| Total protein | -0.185 | 0.025 | -0.221 | 0.007 |
| LDL cholesterol | -0.226 | 0.006 | -0.216 | 0.009 |

Then, the above-described complex regression models were applied. Eventually, the insulin sensitivity index was developed using the 'supervised principal components model which predicted variable M in an optimal way while the number of predicators was reduced (unlike in the ridge and random forest regression models wherein all examined variables are used). On that basis the insulin sensitivity index according to the present invention was developed, and its parameters are included in Table 3 below.

Table 3. Coefficients of the 'supervised principal components' model from the area relating to the clinical characteristics predicting variable M in the best way.

| Variable | Mean | S.D. | PC1 | Coefficient |
|---|---|---|---|---|
| Free text | | | 0.0000 | 11.5847 |
| RBC | 5.0291 | 0.3938 | -0.1457 | -0.9622 |
| AlAt | 24.7243 | 11.894 | -0.1407 | -0.0308 |
| C-peptide | 0.6693 | 0.2777 | -0.1145 | -1.0718 |
| SHBG | 28.6501 | 16.2141 | 0.1493 | 0.0239 |
| Adiponectin | 16.5945 | 7.2721 | 0.1303 | 0.0466 |
| IGFBP-1 | 3.6257 | 3.2367 | 0.1502 | 0.1206 |

S.D. - standard deviation.
PC1 - coefficients of an equation that makes it possible to determine the value of relevant component on the basis of standardized values of predicators.
Coefficient - coefficients of an equation that makes it possible to determine a predicted value of the dependent variable M on the basis of original (non-standardized) values of predicators.

The measures of the quality of the goodness of model fit are presented in Table 4. The coefficient of correlation with variable M for the testing sample was 0.5926; whereas the area under the curve (AUC) of the receiver operating characteristics (ROC) demonstrates that the obtained diagnostic power was 0.80.

Table 4. Measures of the quality of the goodness of fit for the presented model

| Model | Characteristics |
|---|---|
| N variables | 103 |
| N model | 6 |
| $R^2$ learning | 0.3070 |
| R learning | 0.5541 |
| RMSE learning | 2.5357 |
| AUC learning | 0.7172 |
| $R^2$ testing | 0.3511 |
| R testing | 0.5926 |
| RMSE testing | 2.0946 |
| AUC testing | 0.8009 |

N variables - the number of independent variables in the data set used in the construction of the model,
N model - the number of independent variables used in the dependent models,
$R^2$ learning ($R^2$ testing) - coefficient of determination determined in the learning set (respectively testing),
R learning (R testing) - Pearson correlation coefficient (Pearsons's R) between the observed and predicted values of variable in the learning set (respectively testing),
RMSE learning (RMSE testing) - root-mean-square error determined in the learning set (respectively testing),
AUC learning (AUC testing) - AUC value (area under curve ROC) determined in the learning set (respectively testing).

The sensitivity and specificity curves illustrate how the sensitivity and specificity of the test changes depending on the cut-off point. Figure 1 shows a curve for testing sample. For the cut-off point 7.519, the sensitivity of the developed insulin sensitivity index is 90%, whereas specificity is 68%.

*Example 2*

*A comparison of the insulin sensitivity index according to the invention with other methods and indices of insulin sensitivity/insulin resistance.*

Differences between the developed method for determining insulin sensitivity and insulin sensitivity index according to the invention and other methods and indices of insulin sensitivity/insulin resistance known in this field were also studied. A group of human subjects with low insulin sensitivity showed a higher HOMA-IR index (p = 0.0009) and lower QUICKI index (p = 0.0002). The highest statistical significance was observed for the insulin sensitivity index developed by the present inventors - it was significantly lower in the group of human subjects with low insulin sensitivity assessed in the clamp test (p = 0.000001) (Table 5).

Table 5. The developed index and other indices of insulin sensitivity/insulin resistance in the examined groups of human subjects.

| | High insulin sensitivity | Low insulin sensitivity | p |
|---|---|---|---|
| Insulin sensitivity index | 7.57±1.33 | 6.49±1.17 | 0.000001 |
| HOMA-IR | 2.14±1.14 | 2.89±1.57 | 0.0009 |
| QUICKI | 0.15±0.01 | 0.14±0.01 | 0.0002 |
| M (mg/kg FFM/min) | 8.94±2.51 | 4.57±1.04 | 0.000000 |

A correlation of the index developed by the present inventors with the result of insulin resistance obtained in the clamp test was also analyzed and a strong, significant correlation with variable M was demonstrated (r = 0.57, p < 0.000001, Figure 2A), which is stronger than that in the case of HOMA-IR index (r=-0.34, p = 0.00002, Figure 2B) and QUICKI index (r = 0.34, p = 0.00002, Figure 2C). This means that the presented index reflects tissue insulin sensitivity in a better and more precise way.

*Example 3*

*Identifying insulin resistance and determining a predisposition for a disorder connected with insulin resistance*

The method for identifying insulin resistance and determining a predisposition for a disorder connected with insulin resistance was carried out in a volunteer (No. 47) in the way as described above. All tests and analyses were carried out in the way and with the use of kits and analysers as described above. The following measured values of RBC count, AIAT activity, concentration of C-peptide, adiponectin and SHGB and IGFBP1 were obtained: RBC: 5.660 mln/$\mu$l, ALT: 44 U/l, C-peptide: 1.5005 pmol/ml, SHBG: 12.784 nmol/l, Adiponectin: 7.146 $\mu$g/ml, IGFBP1: 0.5841 ng/ml. Then, an insulin sensitivity index according to the invention was calculated in the following way:

Insulin sensitivity index = 11.5847 - 5.660 x 0.9622 - 44 x 0.0308 - 1.5005 x 1.0718 + 12.784 x 0.0239 + 7.146 x 0.0466 + 0.5841 x 0.1206 = 3.884.

The insulin sensitivity index value of 3.884 was obtained, which was considerably below the cut-off point: 7.5, and the existence of insulin resistance and a predisposition of a disorder connected with insulin resistance was diagnosed. **[0114]** This result was then confirmed using the clamp technique test in the way and with the use of kits and analysers as described above. M value of 3.455 mg/kg FFM/min was obtained which proves the existence of insulin resistance in that human subject.
Thus, the above results prove that the invention makes it possible to identify the existence of insulin resistance and a predisposition for disorders connected therewith on the basis of the results of measurements obtained from one blood draw in a sensitive, specific, quick and reliable way.

*Example 4*

*Identifying the lack of insulin resistance and determining the lack of a predisposition for a disorder connected with insulin resistance*

The method for identifying insulin resistance and determining a predisposition for a disorder connected with insulin resistance was carried out in a volunteer (No. 122) in way described above. All tests and analyses were carried out in the way and with the use of kits and analysers as described above. The following measured values of RBC count, AIAT activity, concentration of C-peptide, adiponectin and SHBG and IGFBP1 were obtained: RBC: 4.35 mln/$\mu$l, ALT: 17 U/l, C-peptide: 0.4889 pmol/ml, SHBG: 32.368 nmol/l, Adiponectin: 35.865 $\mu$g/ml, IGFBP1: 7.3432 ng/ml. Then, an insulin sensitivity index according to the invention was calculated in the following way:

Insulin sensitivity index = 11.5847 - 4.35 x 0.9622 - 17 x 0.0308 - 0.4889 x 1.0718 + 32.368 x 0.0239 + 35.865 x 0.0466 + 7.3432 x 0.1206 = 9.763.

The insulin sensitivity index value of 9.763 was obtained, which was considerably above the cut-off point: 7.5, and thus the existence of high insulin sensitivity and the lack of insulin resistance were identified in that human subject.
This result was then confirmed using the test clamp technique test in the way and with the use of kits and analysers as described above. M value of 15.888 mg/kg FFM/min was obtained which evidences the lack of insulin resistance in that human subject.
Thus, the above results prove that the invention makes it possible to determine tissue insulin sensitivity, to determine the existence of insulin resistance and a predisposition for disorders connected therewith on the basis of the results of measurements obtained from one blood draw, in a sensitive, specific, quick and reliable way.

**Claims**

1. A method for determining tissue insulin sensitivity in a human subject, **characterized in that**

   a) in a blood sample drawn from a human subject, the following laboratory parameters and concentrations of the following proteins in blood serum are measured: red blood cells (RBC), alanine aminotransferase (AIAT), C-peptide, sex hormone binding globulin (*SHBG*)*,* adiponectin, insulin-like growth factor binding protein-1 (IGFBP1);
   b) an insulin sensitivity index is determined on the basis of obtained results of measurements specified in a) using the following equation:

Insulin sensitivity index = 11.5847 - RBC x 0.9622 - AIAT x 0.0308 - C-peptide x 1.0718 + SHBG x 0.0239 + Adiponectin x 0.0466 + IGFBP1 x 0.1206,

wherein

RBC is the value of red blood cells count measured in the sample using a method having 4-6 mln/$\mu$l reference interval for general population;
AIAT is the value of alanine aminotransferase activity measured in the sample using a method having 5 - 50 U/l reference interval for general population;
C-peptide is the value of C-peptide concentration measured using a method having 0.003 - 0.04 pmol/ml sensitivity and 0.26 - 2 pmol/ml reference interval for general population;
SHBG is the value of the concentration of sex hormone-binding globulin measured using a method having 0.02 - 0.35 nmol/l sensitivity and 9 - 144 nmol/l reference interval for general population;
Adiponectin is the value of adiponectin concentration measured using a method having 0.15 - 0.5 $\mu$g/ml sensitivity and 6 - 30 $\mu$g/ml reference interval for general population;
IGFBP1 is the value of the concentration of insulin-like growth factor binding protein-1 measured using a method having 0.009 - 0.02 ng/ml sensitivity and 0.2 - 18 ng/ml reference interval for general population.

2. A method for identifying insulin resistance and determining a predisposition for a disorder connected with insulin resistance in a human subject, **characterized in that**

a) in a blood sample drawn from a human subject, the following laboratory parameters and concentrations of the following proteins in blood serum are measured: red blood cells (RBC), alanine aminotransferase (AIAT), C-peptide, sex hormone binding globulin (SHBG), adiponectin, insulin-like growth factor binding protein-1 (IGFBP1);
b) an insulin sensitivity index is determined on the basis of obtained results of measurements specified in a) using the following equation:

Insulin sensitivity index = 11.5847 - RBC x 0.9622 - AIAT x 0.0308 - C-peptide x 1.0718 + SHBG x 0.0239 + Adiponectin x 0.0466 + IGFBP1 x 0.1206,

wherein

RBC is the value of red blood cells count measured in the sample using a method having 4-6 mln/$\mu$l reference interval for general population;
AIAT is the value of alanine aminotransferase activity measured in the sample using a method having 5 - 50 U/l reference interval for general population;
C-peptide is the value of C-peptide concentration measured using a method having 0.003 - 0.04 pmol/ml sensitivity and 0.26 - 2 pmol/ml reference interval for general population;
SHBG is the value of the concentration of sex hormone-binding globulin measured using a method having 0.02 - 0.35 nmol/l sensitivity and 9 - 144 nmol/l reference interval for general population;
Adiponectin is the value of adiponectin concentration measured using a method having 0.15 - 0.5 $\mu$g/ml sensitivity and 6-30 $\mu$g/ml reference interval for general population;
IGFBP1 is the value of the concentration of insulin-like growth factor binding protein-1 measured using a method having 0.009 - 0.02 ng/ml sensitivity and 0.2 - 18 ng/ml reference interval for general population;

c) insulin resistance is identified and a predisposition for a disorder connected with insulin resistance is determined when the obtained value of insulin sensitivity index is 7.5 or less.

3. The method according to claim 1, **characterized in that** the method is further for the prognosis, diagnosis, including early diagnosis, or therapy monitoring of a disorder connected with insulin resistance.

4. Use of insulin sensitivity index defined by the following equation

Insulin sensitivity index = 11.5847 - RBC x 0.9622 - AIAT x 0.0308 - C-peptide x 1.0718 + SHBG x 0.0239 + Adiponectin x 0.0466 + IGFBP1 x 0.1206,

wherein

RBC is the value of red blood cells count measured in the sample using a method having 4-6 mln/$\mu$l reference interval for general population;

AlAT is the value of alanine aminotransferase activity measured in the sample using a method having 5 - 50 U/l reference interval for general population;

C-peptide is the value of C-peptide concentration measured using a method having 0.003 - 0.04 pmol/ml sensitivity and 0.26 - 2 pmol/ml reference interval for general population;

SHBG is the value of the concentration of sex hormone-binding globulin measured using a method having 0.02 - 0.35 nmol/l sensitivity and 9 - 144 nmol/l reference interval for general population;

Adiponectin is the value of adiponectin concentration measured using a method having 0.15 - 0.5 $\mu$g/ml sensitivity and 6-30 $\mu$g/ml reference interval for general population;

IGFBP1 is the value of the concentration of insulin-like growth factor binding protein-1 measured using a method having 0.009 - 0.02 ng/ml sensitivity and 0.2 - 18 ng/ml reference interval for general population;

as an insulin resistance biomarker.

5. Use of insulin sensitivity index defined by the following equation

Insulin sensitivity index = 11.5847 - RBC x 0.9622 - AlAT x 0.0308 - C-peptide x 1.0718 + SHBG x 0.0239 + Adiponectin x 0.0466 + IGFBP1 x 0.1206,

wherein

RBC is the value of red blood cells count measured in the sample using a method having 4-6 mln/$\mu$l reference interval for general population;

AlAT is the value of alanine aminotransferase activity measured in the sample using a method having 5 - 50 U/l reference interval for general population;

C-peptide is the value of C-peptide concentration measured using a method having 0.003 - 0.04 pmol/ml sensitivity and 0.26 - 2 pmol/ml reference interval for general population;

SHBG is the value of the concentration of sex hormone-binding globulin measured using a method having 0.02 - 0.35 nmol/l sensitivity and 9 - 144 nmol/l reference interval for general population;

Adiponectin is the value of adiponectin concentration measured using a method having 0.15 - 0.5 $\mu$g/ml sensitivity and 6-30 $\mu$g/ml reference interval for general population;

IGFBP1 is the value of the concentration of insulin-like growth factor binding protein-1 measured using a method having 0.009 - 0.02 ng/ml sensitivity and 0.2 - 18 ng/ml reference interval for general population;

as a prognostic biomarker of a disorder connected with insulin resistance.

6. Use of insulin sensitivity index defined by the following equation

Insulin sensitivity index = 11.5847 - RBC x 0.9622 - AlAT x 0.0308 - C-peptide x 1.0718 + SHBG x 0.0239 + Adiponectin x 0.0466 + IGFBP1 x 0.1206,

wherein

RBC is the value of red blood cells count measured in the sample using a method having 4-6 mln/$\mu$l reference interval for general population;

AlAT is the value of alanine aminotransferase activity measured in the sample using a method having 5 - 50 U/l reference interval for general population;

C-peptide is the value of C-peptide concentration measured using a method having 0.003 - 0.04 pmol/ml sensitivity and 0.26 - 2 pmol/ml reference interval for general population;

SHBG is the value of the concentration of sex hormone-binding globulin measured using a method having 0.02 - 0.35 nmol/l sensitivity and 9 - 144 nmol/l reference interval for general population;

Adiponectin is the value of adiponectin concentration measured using a method having 0.15 - 0.5 $\mu$g/ml sensitivity and 6-30 $\mu$g/ml reference interval for general population;

IGFBP1 is the value of the concentration of insulin-like growth factor binding protein-1 measured using a method having 0.009 - 0.02 ng/ml sensitivity and 0.2 - 18 ng/ml reference interval for general population;

for determining a predisposition for a disorder connected with insulin resistance.

7. Use of an insulin sensitivity index defined by the following equation:

Insulin sensitivity index = 11.5847 - RBC x 0.9622 - AlAT x 0.0308 - C-peptide x 1.0718 + SHBG x 0.0239 + Adiponectin x 0.0466 + IGFBP1 x 0.1206,

wherein

RBC is the value of red blood cells count measured in the sample using a method having 4-6 mln/$\mu$l reference interval for general population;

AlAT is the value of alanine aminotransferase activity measured in the sample using a method having 5 - 50 U/l reference interval for general population;

C-peptide is the value of C-peptide concentration measured using a method having 0.003 - 0.04 pmol/ml sensitivity and 0.26 - 2 pmol/ml reference interval for general population;

SHBG is the value of the concentration of sex hormone-binding globulin measured using a method having 0.02 - 0.35 nmol/l sensitivity and 9 - 144 nmol/l reference interval for general population;

Adiponectin is the value of adiponectin concentration measured using a method having 0.15 - 0.5 $\mu$g/ml sensitivity and 6-30 $\mu$g/ml reference interval for general population;

IGFBP1 is the value of the concentration of insulin-like growth factor binding protein-1 measured using a method having 0.009 - 0.02 ng/ml sensitivity and 0.2 - 18 ng/ml reference interval for general population;

for monitoring tissue insulin sensitivity, in particular in a disorder and/or a disease connected with insulin resistance, in particular type 2 diabetes.

8. The method according to any one of claims 1 - 3, or use according to any one of claims 4 - 7, **characterized in that** RBC count is measured using a cytometry method with optical laser light scattering analysis and/or an impedance method, AlAT activity is measured using a *kinetic spectrophotometric* method or a calorimetric method, C-peptide concentration is measured using ELISA, RIA method or a chemiluminescence method, SHBG concentration is measured using IRMA, ELISA or a chemiluminescence method, adiponectin concentration is measured using RIA or ELISA method, and IGFBP1 concentration is measured using ELISA or RIA method.

9. The method or use according to claim 8, **characterized in that** RBC count is measured using a cytometry method with optical laser light scattering analysis, AlAT activity is measured using a *kinetic spectrophotometric* method, C-peptide and IGFBP1 concentration is measured using ELISA method, SHBG concentration is measured using IRMA method, and adiponectin concentration is measured using RIA method.

10. The method according to any one of claims 2 - 3 or 8 - 9, or use according to any one of claims 4-9, **characterized in that** a disorder connected with insulin resistance is selected from a group comprising: overweight, obesity, impaired glucose tolerance, dyslipidaemia, metabolic syndrome, fatty liver, polycystic ovary syndrome, arterial hypertension, atherosclerosis, ischaemic heart disease, cerebral stroke, neurodegenerative diseases, in particular type 2 diabetes.

11. The method according to any one of claims 1 - 3 or 8 - 10, or use according to any one of claims 4 - 10, **characterized in that** a human subject is selected from a group comprising: a person with normal body weight, an overweight person, an obese person, a person with increased fasting glycaemia, a person with impaired glucose tolerance, a person with type 2 diabetes, a person with dyslipidaemia, a person with metabolic syndrome, a person with fatty liver, a woman with polycystic ovary syndrome, a person with arterial hypertension, a person with atherosclerosis, a person with ischaemic heart disease, a person after cerebral stroke, a person with a neurodegenerative disease, a woman who had gestational diabetes or gave birth to a baby having over 4 kg body weight, and a first-degree relative of a person suffering from diabetes and/or cardio-vascular diseases, and a person before bariatric surgery and/or after such surgery.

**Patentansprüche**

1. Verfahren zur Bestimmung der Gewebeinsulinempfindlichkeit bei einem menschlichen Probanden, **dadurch gekennzeichnet, dass**

a) in einer einem menschlichen Probanden entnommenen Blutprobe die folgenden Laborparameter und Konzentrationen der folgenden Proteine im Blutserum: rote Blutkörperchen (RBC), Alaninaminotransferase (AlAT), C-Peptid, sexualhormonbindendes Globulin (SHBG), Adiponektin, insulinähnliches Wachstumsfaktor-bindendes Protein-1 (IGFBP1), gemessen werden

b) ein Insulinsensitivitätsindex auf der Grundlage der erhaltenen Ergebnisse der unter a) angegebenen Messungen unter Verwendung der folgenden Gleichung:

Insulinsensitivitätsindex = 11,5847 - RBC x 0,9622 - AlAT x 0,0308 - C-Peptid x 1,0718 + SHBG x 0,0239 + Adiponektin x 0,0466 + IGFBP1 x 0,1206, bestimmt wird,
wobei

RBC der Wert der Anzahl der roten Blutkörperchen ist, der in der Probe unter Verwendung einer Methode mit einem Referenzintervall von 4 - 6 Mio./$\mu$l für die Allgemeinbevölkerung gemessen wurde;
AlAT der Wert der Alaninaminotransferaseaktivität ist, der in der Probe unter Verwendung einer Methode mit einem Referenzintervall von 5 - 50 U/l für die Allgemeinbevölkerung gemessen wurde;
C-Peptid der Wert der C-Peptid-Konzentration ist, der mit einer Methode mit 0,003 - 0,04 pmol/ml Sensitivität und 0,26 - 2 pmol/ml Referenzintervall für die Allgemeinbevölkerung gemessen wurde;
SHBG der Wert der Konzentration des sexualhormonbindenden Globulins ist, der mit einer Methode mit 0,02 - 0,35 nmol/l Sensitivität und 9 - 144 nmol/l Referenzintervall für die Allgemeinbevölkerung gemessen wurde;
Adiponektin der Wert der Adiponektinkonzentration ist, der mit einer Methode mit 0,15 - 0,5 $\mu$g/ml Sensitivität und 6 - 30 $\mu$g/ml Referenzintervall für die Allgemeinbevölkerung gemessen wurde;
IGFBP1 der Wert der Konzentration des insulinähnlichen wachstumsfaktorbindenden Proteins-1 ist, der mit einer Methode mit 0,009 - 0,02 ng/ml Sensitivität und 0,2 - 18 ng/ml Referenzintervall für die Allgemeinbevölkerung gemessen wurde.

2. Verfahren zur Erkennung einer Insulinresistenz und zur Bestimmung einer Prädisposition für eine mit Insulinresistenz verbundene Erkrankung bei einem menschlichen Probanden, **dadurch gekennzeichnet, dass**

a) in einer einem menschlichen Probanden entnommenen Blutprobe die folgenden Laborparameter und Konzentrationen der folgenden Proteine: rote Blutkörperchen (RBC), Alanin-Aminotransferase (AlAT), C-Peptid, sexualhormonbindendes Globulin (SHBG), Adiponektin, insulinähnliches wachstumsfaktorbindendes Protein-1 (IGFBP1), im Blutserum gemessen werden;
b) ein Insulinsensitivitätsindex auf der Grundlage der erhaltenen Ergebnisse der unter a) angegebenen Messungen unter Verwendung der folgenden Gleichung:

Insulinsensitivitätsindex = 11,5847 - RBC x 0,9622 - AlAT x 0,0308 - C-Peptid x 1,0718 + SHBG x 0,0239 + Adiponektin x 0,0466 + IGFBP1 x 0,1206, bestimmt wird,
wobei

RBC der Wert der Anzahl der roten Blutkörperchen ist, der in der Probe unter Verwendung einer Methode mit einem Referenzintervall von 4 - 6 Mio./$\mu$l für die Allgemeinbevölkerung gemessen wurde;
AlAT der Wert der Alaninaminotransferaseaktivität ist, der in der Probe unter Verwendung einer Methode mit einem Referenzintervall von 5 - 50 U/l für die Allgemeinbevölkerung gemessen wurde;
C-Peptid der Wert der C-Peptidkonzentration ist, der mit einer Methode mit 0,003 - 0,04 pmol/ml Sensitivität und 0,26 - 2 pmol/ml Referenzintervall für die Allgemeinbevölkerung gemessen wurde;
SHBG der Wert der Konzentration des sexualhormonbindenden Globulins ist, der mit einer Methode mit 0,02 - 0,35 nmol/l Sensitivität und 9 - 144 nmol/l Referenzintervall für die Allgemeinbevölkerung gemessen wurde;
Adiponektin der Wert der Adiponektinkonzentration ist, der mit einer Methode mit 0,15 - 0,5 $\mu$g/ml Sensitivität und 6 - 30 $\mu$g/ml Referenzintervall für die Allgemeinbevölkerung gemessen wurde;
IGFBP1 der Wert der Konzentration des insulinähnlichen wachstumsfaktorbindenden Proteins-1 ist, der mit einer Methode mit 0,009 - 0,02 ng/ml Sensitivität und 0,2 - 18 ng/ml Referenzintervall für die Allgemeinbevölkerung gemessen wurde;

c) eine Insulinresistenz festgestellt wird und eine Prädisposition für eine mit Insulinresistenz verbundene Erkrankung bestimmt wird, wenn der erhaltene Wert des Insulinsensitivitätsindex 7,5 oder weniger beträgt.

3. Verfahren nach Anspruch 1, **dadurch gekennzeichnet, dass** das Verfahren weiterhin zur Prognose, Diagnose, einschließlich Frühdiagnose, oder Therapieüberwachung einer mit Insulinresistenz verbundenen Erkrankung dient.

4.  Verwendung des Insulinsensitivitätsindex, der durch die folgende Gleichung:

Insulinsensitivitätsindex = 11,5847 - RBC x 0,9622 - AlAT x 0,0308 - C-Peptid x 1,0718 + SHBG x 0,0239 + Adiponektin x 0,0466 + IGFBP1 x 0,1206,

definiert ist,
wobei

RBC der Wert der Anzahl der roten Blutkörperchen ist, der in der Probe mit einer Methode, die einen Referenzbereich von 4 - 6 Mio./µl für die Allgemeinbevölkerung hat, gemessen wurde;
AlAT der Wert der Alaninaminotransferaseaktivität ist, der in der Probe mit einer Methode, die einen Referenzbereich von 5 - 50 U/l für die Allgemeinbevölkerung hat, gemessen wurde;
C-Peptid der Wert der C-Peptidkonzentration ist, der mit einer Methode, die eine Sensitivität von 0,003 - 0,04 pmol/ml und einen Referenzbereich von 0,26 - 2 pmol/ml für die Allgemeinbevölkerung hat, gemessen wurde;
SHBG der Wert der Konzentration des sexualhormonbindenden Globulins ist, der mit einer Methode mit 0,02 - 0,35 nmol/l Sensitivität und 9 - 144 nmol/l Referenzintervall für die Allgemeinbevölkerung, gemessen wurde;
Adiponektin der Wert der Adiponektinkonzentration ist, der mit einer Methode mit 0,15 - 0,5 µg/ml Sensitivität und 6 - 30 µg/ml Referenzintervall für die Allgemeinbevölkerung, gemessen wurde;
IGFBP1 der Wert der Konzentration des insulinähnlichen wachstumsfaktorbindenden Proteins-1 ist, der mit einer Methode mit 0,009 - 0,02 ng/ml Sensitivität und 0,2 - 18 ng/ml Referenzintervall für die Allgemeinbevölkerung gemessen wurde;
als Biomarker für Insulinresistenz.

5.  Verwendung des Insulinsensitivitätsindex, der durch die folgende Gleichung:

Insulinsensitivitätsindex = 11,5847 - RBC x 0,9622 - AlAT x 0,0308 - C-Peptid x 1,0718 + SHBG x 0,0239 + Adiponektin x 0,0466 + IGFBP1 x 0,1206,

definiert ist,
wobei

RBC der Wert der Anzahl der roten Blutkörperchen ist, der in der Probe mit einer Methode, die für die Allgemeinbevölkerung einen Referenzbereich von 4 - 6 Mio./µl hat, gemessen wurde;
AlAT der Wert der Alaninaminotransferaseaktivität ist, der in der Probe mit einer Methode, die für die Allgemeinbevölkerung einen Referenzbereich von 5 - 50 U/l hat, gemessen wurde;
C-Peptid der Wert der C-Peptidkonzentration ist, der mit einer Methode, die für die Allgemeinbevölkerung eine Sensitivität von 0,003 - 0,04 pmol/ml und einen Referenzbereich von 0,26 - 2 pmol/ml hat, gemessen wurde;
SHBG der Wert der Konzentration des sexualhormonbindenden Globulins ist, der mit einer Methode mit einer Sensitivität von 0,02 - 0,35 nmol/l und einem Referenzintervall von 9 - 144 nmol/l für die Allgemeinbevölkerung, gemessen wurde;
Adiponektin ist der Wert der Adiponektinkonzentration ist, der mit einer Methode mit einer Sensitivität von 0,15 - 0,5 µg/ml und einem Referenzintervall von 6 - 30 µg/ml für die Allgemeinbevölkerung, gemessen wurde;
IGFBP1 der Wert der Konzentration des insulinähnlichen wachstumsfaktorbindenden Proteins-1 ist, der mit einer Methode mit einer Sensitivität von 0,009 - 0,02 ng/ml und einem Referenzintervall von 0,2 - 18 ng/ml für die Allgemeinbevölkerung, gemessen wurde;
als prognostischer Biomarker einer mit Insulinresistenz verbundenen Störung.

6.  Verwendung des Insulinsensitivitätsindex, der durch die folgende Gleichung:

Insulinsensitivitätsindex = 11,5847 - RBC x 0,9622 - AlAT x 0,0308 - C-Peptid x 1,0718 + SHBG x 0,0239 + Adiponektin x 0,0466 + IGFBP1 x 0,1206,

definiert ist,
wobei

RBC der Wert der Anzahl der roten Blutkörperchen ist, der in der Probe mit einer Methode, die für die Allgemeinbevölkerung einen Referenzbereich von 4 - 6 Mio./µl hat, gemessen wurde;

AIAT der Wert der Alaninaminotransferaseaktivität ist, der in der Probe mit einer Methode, die für die Allgemeinbevölkerung einen Referenzbereich von 5 - 50 U/l hat, gemessen wurde;

C-Peptid der Wert der C-Peptidkonzentration ist, der mit einer Methode, die für die Allgemeinbevölkerung eine Sensitivität von 0,003 - 0,04 pmol/ml und einen Referenzbereich von 0,26 - 2 pmol/ml hat, gemessen wurde;

SHBG der Wert der Konzentration des sexualhormonbindenden Globulins ist, der mit einer Methode mit 0,02 - 0,35 nmol/l Sensitivität und 9 - 144 nmol/l Referenzintervall für die Allgemeinbevölkerung gemessen wurde;

Adiponektin der Wert der Adiponektinkonzentration ist, der mit einer Methode mit 0,15 - 0,5 µg/ml Sensitivität und 6 - 30 µg/ml Referenzintervall für die Allgemeinbevölkerung gemessen wurde;

IGFBP1 der Wert der Konzentration des insulinähnlichen wachstumsfaktorbindenden Proteins-1 ist, der mit einer Methode mit 0,009 - 0,02 ng/ml Sensitivität und 0,2 - 18 ng/ml Referenzintervall für die Allgemeinbevölkerung, gemessen wurde;

zur Bestimmung einer Prädisposition für eine mit Insulinresistenz verbundene Erkrankung.

7. Verwendung eines Insulinsensitivitätsindex, der durch die folgende Gleichung:

Insulinsensitivitätsindex = 11,5847 - RBC x 0,9622 - AIAT x 0,0308 - C-Peptid x 1,0718 + SHBG x 0,0239 + Adiponektin x 0,0466 + IGFBP1 x 0,1206

definiert ist,
wobei

RBC der Wert der Anzahl der roten Blutkörperchen ist, der in der Probe mit einer Methode, die einen Referenzbereich von 4 - 6 Mio./µl für die Allgemeinbevölkerung hat, gemessen wurde;

AIAT der Wert der Alaninaminotransferaseaktivität ist, der in der Probe mit einer Methode, die einen Referenzbereich von 5 - 50 U/l für die Allgemeinbevölkerung hat, gemessen wurde;

C-Peptid der Wert der C-Peptidkonzentration ist, der mit einer Methode, die eine Sensitivität von 0,003 - 0,04 pmol/ml und einen Referenzbereich von 0,26 - 2 pmol/ml für die Allgemeinbevölkerung hat, gemessen wurde;

SHBG der Wert der Konzentration des sexualhormonbindenden Globulins ist, der mit einer Methode mit 0,02 - 0,35 nmol/l Sensitivität und 9 - 144 nmol/l Referenzintervall für die Allgemeinbevölkerung gemessen wurde;

Adiponektin der Wert der Adiponektinkonzentration ist, der mit einer Methode mit 0,15 - 0,5 µg/ml Sensitivität und 6 - 30 µg/ml Referenzintervall für die Allgemeinbevölkerung gemessen wurde;

IGFBP1 der Wert der Konzentration des insulinähnlichen wachstumsfaktorbindenden Proteins-1 ist, der mit einer Methode mit 0,009 - 0,02 ng/ml Sensitivität und 0,2 - 18 ng/ml Referenzintervall für die Allgemeinbevölkerung gemessen wurde;

zur Überwachung der Gewebeinsulinsensitivität, insbesondere bei einer Störung und/oder einer Krankheit, die mit Insulinresistenz verbunden ist, insbesondere des Typ-2-Diabetes.

8. Verfahren nach einem der Ansprüche 1-3 oder Verwendung nach einem der Ansprüche 4-7, **dadurch gekennzeichnet, dass** die Anzahl der RBC unter Verwendung einer Zytometriemethode mit optischer Laserlichtstreuungsanalyse und/oder einer Impedanzmethode gemessen wird, die AIAT-Aktivität unter Verwendung einer kinetischen spektrophotometrischen Methode oder einer kalorimetrischen Methode gemessen wird, die C-Peptidkonzentration unter Verwendung einer ELISA-, RIA-Methode oder einer Chemilumineszenzmethode gemessen wird, die SHBG-Konzentration unter Verwendung einer IRMA-, ELISA- oder Chemilumineszenzmethode gemessen wird, die Adiponektinkonzentration unter Verwendung einer RIA- oder ELISA-Methode gemessen wird und die IGFBP1-Konzentration unter Verwendung einer ELISA- oder RIA-Methode gemessen wird.

9. Verfahren oder Verwendung nach Anspruch 8, **dadurch gekennzeichnet, dass** die Anzahl der RBC unter Verwendung einer Zytometriemethode mit optischer Laserlichtstreuanalyse gemessen wird, die AIAT-Aktivität unter Verwendung einer kinetischen spektrophotometrischen Methode gemessen wird, die C-Peptid- und IGFBP1-Konzentrationen unter Verwendung der ELISA-Methode gemessen werden, die SHBG-Konzentration unter Verwendung der IRMA-Methode gemessen wird und die AdiponektinKonzentration unter Verwendung der RIA-Methode gemessen wird.

10. Verfahren nach einem der Ansprüche 2-3 oder 8-9 oder Verwendung nach einem der Ansprüche 4-9, **dadurch gekennzeichnet, dass** eine mit Insulinresistenz verbundene Störung aus einer Gruppe die das Übergewicht, Fettleibigkeit, gestörte Glukosetoleranz, Dyslipidämie, metabolisches Syndrom, Fettleber, polyzystisches Ovarialsyndrom, arterielle Hypertonie, Arteriosklerose, ischämische Herzkrankheit, Schlaganfall, neurodegenerative Erkrankungen, insbesondere Typ-2-Diabetes, umfasst, ausgewählt wird.

11. Verfahren nach einem der Ansprüche 1-3 oder 8-10 oder Verwendung nach einem der Ansprüche 4-10, **dadurch gekennzeichnet, dass** ein menschliches Subjekt aus einer Gruppe, die eine Person mit normalem Körpergewicht, eine übergewichtige Person, eine adipöse Person, eine Person mit erhöhtem Nüchtern-Glykämie, eine Person mit gestörter Glukosetoleranz, eine Person mit Typ-2-Diabetes, eine Person mit Dyslipidämie, eine Person mit metabolischem Syndrom, eine Person mit Fettleber, eine Frau mit polyzystischem Ovarialsyndrom, eine Person mit arterieller Hypertonie, eine Person mit Arteriosklerose, eine Person mit ischämischer Herzkrankheit, eine Person nach einem Schlaganfall, eine Person mit einer neurodegenerativen Erkrankung, eine Frau, die Schwangerschaftsdiabetes hatte oder ein Baby mit über 4 kg Körpergewicht zur Welt gebracht hat, und einen Verwandten ersten Grades einer Person, die an Diabetes und/oder Herz-Kreislauf-Erkrankungen leidet, und eine Person vor und/oder nach einer bariatrischen Operation umfasst, ausgewählt ist.

**Revendications**

1. Procédé de détermination de la sensibilité tissulaire à l'insuline chez un sujet humain, **caractérisé en ce que**

a) dans un échantillon de sang prélevé sur un sujet humain, les paramètres de laboratoire et les concentrations des protéines suivantes dans le sérum sanguin sont mesurés : globules rouges (RBC), alanine aminotransférase (AlAT), peptide C, globuline liant des hormones sexuelles (SHBG), adiponectine, protéine-1 liant du facteur de croissance analogue à l'insuline (IGFBP1) ;
b) un indice de sensibilité à l'insuline est déterminé sur la base des résultats obtenus des mesures spécifiées en a) en utilisant l'équation suivante :

Indice de sensibilité à l'insuline = 11,5847 - RBC x 0,9622 - AlAT x 0,0308 - Peptide C x 1,0718 + SHBG x 0,0239 + Adiponectine x 0,0466 + IGFBP1 x 0,1206, où

RBC est la valeur du nombre de globules rouges mesurée dans l'échantillon en utilisant une méthode ayant un intervalle de référence de 4 à 6 mln/µl pour la population générale ;
AlAT est la valeur de l'activité d'alanine aminotransférase mesurée dans l'échantillon en utilisant une méthode ayant un intervalle de référence de 5 à 50 U/l pour la population générale ;
Peptide C est la valeur de la concentration du peptide C mesurée à l'aide d'une méthode ayant une sensibilité de 0,003 à 0,04 pmol/ml et un intervalle de référence de 0,26 à 2 pmol/ml pour la population générale ;
SHBG est la valeur de la concentration de la globuline liant les hormones sexuelles mesurée à l'aide d'une méthode ayant une sensibilité de 0,02 à 0,35 nmol/l et un intervalle de référence de 9 à 144 nmol/l pour la population générale ;
Adiponectine est la valeur de la concentration d'adiponectine mesurée à l'aide d'une méthode ayant une sensibilité de 0,15 à 0,5 µg/ml et un intervalle de référence de 6 à 30 µg/ml pour la population générale ;
IGFBP1 est la valeur de la concentration de la protéine de liaison du facteur de croissance analogue à l'insuline-1 mesurée à l'aide d'une méthode ayant une sensibilité de 0,009 à 0,02 ng/ml et un intervalle de référence de 0,2 à 18 ng/ml pour la population générale.

2. Procédé d'identification de la résistance à l'insuline et de détermination d'une prédisposition à un trouble lié à la résistance à l'insuline chez un sujet humain, **caractérisé en ce que**

a) dans un échantillon de sang prélevé sur un sujet humain, les paramètres de laboratoire et les concentrations des protéines suivantes dans le sérum sanguin sont mesurés : globules rouges (RBC), alanine aminotransférase (AlAT), peptide C, globuline liant des hormones sexuelles (SHBG), adiponectine, protéine-1 liant du facteur de croissance analogue à l'insuline (IGFBP1) ;
b) un indice de sensibilité à l'insuline est déterminé sur la base des résultats obtenus des mesures spécifiées au point a) en utilisant l'équation suivante :

Indice de sensibilité à l'insuline = 11,5847 - RBC x 0,9622 - AlAT x 0,0308 - Peptide C x 1,0718 + SHBG x 0,0239 + Adiponectine x 0,0466 + IGFBP1 x 0,1206, où

RBC est la valeur du nombre de globules rouges mesurée dans l'échantillon en utilisant une méthode ayant un intervalle de référence de 4 à 6 mln/µl pour la population générale ;

AlAT est la valeur de l'activité d'alanine aminotransférase mesurée dans l'échantillon en utilisant une méthode ayant un intervalle de référence de 5 à 50 U/l pour la population générale ;

Peptide C est la valeur de la concentration en peptide C mesurée à l'aide d'une méthode ayant une sensibilité de 0,003 à 0,04 pmol/ml et un intervalle de référence de 0,26 à 2 pmol/ml pour la population générale ;

SHBG est la valeur de la concentration en globuline liant les hormones sexuelles mesurée à l'aide d'une méthode ayant une sensibilité de 0,02 à 0,35 nmol/l et un intervalle de référence de 9 à 144 nmol/l pour la population générale ;

Adiponectine est la valeur de la concentration en adiponectine mesurée à l'aide d'une méthode ayant une sensibilité de 0,15 à 0,5 µg/ml et un intervalle de référence de 6 à 30 µg/ml pour la population générale ;

IGFBP1 est la valeur de la concentration en protéine-1 liant du facteur de croissance analogue à l'insuline mesurée à l'aide d'une méthode ayant une sensibilité de 0,009 à 0,02 ng/ml et un intervalle de référence de 0,2 à 18 ng/ml pour la population générale ;

c) la résistance à l'insuline est identifiée et une prédisposition à un trouble lié à la résistance à l'insuline est déterminée lorsque la valeur obtenue de l'indice de sensibilité à l'insuline est de 7,5 ou moins.

3. Procédé selon la revendication 1, **caractérisé en ce que** le procédé est en outre destiné au pronostic, au diagnostic, y compris au diagnostic précoce, ou au suivi thérapeutique d'un trouble lié à la résistance à l'insuline.

4. Utilisation de l'indice de sensibilité à l'insuline défini par l'équation suivante Indice de sensibilité à l'insuline = 11,5847 - RBC x 0,9622 - AlAT x 0,0308 - Peptide C x 1,0718 + SHBG x 0,0239 + Adiponectine x 0,0466 + IGFBP1 x 0,1206, où

RBC est la valeur du nombre de globules rouges mesurée dans l'échantillon à l'aide d'une méthode ayant un intervalle de référence de 4 à 6 mln/µl pour la population générale ;

AlAT est la valeur de l'activité d'alanine aminotransférase mesurée dans l'échantillon à l'aide d'une méthode ayant un intervalle de référence de 5 à 50 U/l pour la population générale ;

Peptide C est la valeur de la concentration de peptide C mesurée à l'aide d'une méthode ayant une sensibilité de 0,003 à 0,04 pmol/ml et un intervalle de référence de 0,26 à 2 pmol/ml pour la population générale ;

SHBG est la valeur de la concentration de globuline liant les hormones sexuelles mesurée à l'aide d'une méthode ayant une sensibilité de 0,02 à 0,35 nmol/l et un intervalle de référence de 9 à 144 nmol/l pour la population générale ;

Adiponectine est la valeur de la concentration d'adiponectine mesurée à l'aide d'une méthode ayant une sensibilité de 0,15 à 0,5 µg/ml et un intervalle de référence de 6 à 30 µg/ml pour la population générale ;

IGFBP1 est la valeur de la concentration de protéine-1 liant le facteur de croissance analogue à l'insuline mesurée à l'aide d'une méthode ayant une sensibilité de 0,009 à 0,02 ng/ml et un intervalle de référence de 0,2 à 18 ng/ml pour la population générale ;

comme un biomarqueur de la résistance à l'insuline.

5. Utilisation de l'indice de sensibilité à l'insuline défini par l'équation suivante Indice de sensibilité à l'insuline = 11,5847 - RBC x 0,9622 - AlAT x 0,0308 - Peptide C x 1,0718 + SHBG x 0,0239 + Adiponectine x 0,0466 + IGFBP1 x 0,1206, où

RBC est la valeur du nombre de globules rouges mesurée dans l'échantillon à l'aide d'une méthode ayant un intervalle de référence de 4 à 6 mln/µl pour la population générale ;

AlAT est la valeur de l'activité d'alanine aminotransférase mesurée dans l'échantillon à l'aide d'une méthode ayant un intervalle de référence de 5 à 50 U/l pour la population générale ;

Peptide C est la valeur de la concentration de peptide C mesurée à l'aide d'une méthode ayant une sensibilité de 0,003 à 0,04 pmol/ml et un intervalle de référence de 0,26 à 2 pmol/ml pour la population générale ;

SHBG est la valeur de la concentration de globuline liant les hormones sexuelles mesurée à l'aide d'une méthode ayant une sensibilité de 0,02 à 0,35 nmol/l et un intervalle de référence de 9 à 144 nmol/l pour la population générale ;

Adiponectine est la valeur de la concentration d'adiponectine mesurée à l'aide d'une méthode ayant une sensibilité de 0,15 à 0,5 μg/ml et un intervalle de référence de 6 à 30 μg/ml pour la population générale ;

IGFBP1 est la valeur de la concentration de protéine-1 liant du facteur de croissance analogue à l'insuline mesurée à l'aide d'une méthode ayant une sensibilité de 0,009 à 0,02 ng/ml et un intervalle de référence de 0,2 à 18 ng/ml pour la population générale ;

comme un biomarqueur pronostique d'un trouble lié à la résistance à l'insuline.

6. Utilisation de l'indice de sensibilité à l'insuline défini par l'équation suivante Indice de sensibilité à l'insuline = 11,5847 - RBC x 0,9622 - AlAT x 0,0308 - Peptide C x 1,0718 + SHBG x 0,0239 + Adiponectine x 0,0466 + IGFBP1 x 0,1206, où

RBC est la valeur du nombre de globules rouges mesurée dans l'échantillon à l'aide d'une méthode ayant un intervalle de référence de 4 à 6 mln/μl pour la population générale ;

AlAT est la valeur de l'activité d'alanine aminotransférase mesurée dans l'échantillon à l'aide d'une méthode ayant un intervalle de référence de 5 à 50 U/l pour la population générale ;

Peptide C est la valeur de la concentration de peptide C mesurée à l'aide d'une méthode ayant une sensibilité de 0,003 à 0,04 pmol/ml et un intervalle de référence de 0,26 à 2 pmol/ml pour la population générale ;

SHBG est la valeur de la concentration de globuline liant les hormones sexuelles mesurée à l'aide d'une méthode ayant une sensibilité de 0,02 à 0,35 nmol/l et un intervalle de référence de 9 à 144 nmol/l pour la population générale ;

Adiponectine est la valeur de la concentration d'adiponectine mesurée à l'aide d'une méthode ayant une sensibilité de 0,15 à 0,5 μg/ml et un intervalle de référence de 6 à 30 μg/ml pour la population générale ;

IGFBP1 est la valeur de la concentration de protéine-1 liant du facteur de croissance analogue à l'insuline mesurée à l'aide d'une méthode ayant une sensibilité de 0,009 à 0,02 ng/ml et un intervalle de référence de 0,2 à 18 ng/ml pour la population générale ;

pour déterminer une prédisposition à un trouble lié à la résistance à l'insuline.

7. Utilisation d'un indice de sensibilité à l'insuline défini par l'équation suivante Indice de sensibilité à l'insuline = 11,5847 - RBC x 0,9622 - AlAT x 0,0308 - Peptide C x 1,0718 + SHBG x 0,0239 + Adiponectine x 0,0466 + IGFBP1 x 0,1206, où

RBC est la valeur du nombre de globules rouges mesurée dans l'échantillon à l'aide d'une méthode ayant un intervalle de référence de 4 à 6 mln/μl pour la population générale ;

AlAT est la valeur de l'activité d'alanine aminotransférase mesurée dans l'échantillon à l'aide d'une méthode ayant un intervalle de référence de 5 à 50 U/l pour la population générale ;

Peptide C est la valeur de la concentration de peptide C mesurée à l'aide d'une méthode ayant une sensibilité de 0,003 à 0,04 pmol/ml et un intervalle de référence de 0,26 à 2 pmol/ml pour la population générale ;

SHBG est la valeur de la concentration de globuline liant les hormones sexuelles mesurée à l'aide d'une méthode ayant une sensibilité de 0,02 à 0,35 nmol/l et un intervalle de référence de 9 à 144 nmol/l pour la population générale ;

Adiponectine est la valeur de la concentration d'adiponectine mesurée à l'aide d'une méthode ayant une sensibilité de 0,15 à 0,5 μg/ml et un intervalle de référence de 6 à 30 μg/ml pour la population générale ;

IGFBP1 est la valeur de la concentration de protéine-1 liant le facteur de croissance analogue à l'insuline mesurée à l'aide d'une méthode ayant une sensibilité de 0,009 à 0,02 ng/ml et un intervalle de référence de 0,2 à 18 ng/ml pour la population générale ;

pour surveiller la sensibilité tissulaire à l'insuline, en particulier dans un trouble et/ou une maladie liée à la résistance à l'insuline, en particulier le diabète de type 2.

**8.** Procédé selon l'une quelconque des revendications 1 à 3, ou utilisation selon l'une quelconque des revendications 4 à 7, **caractérisé en ce que** le nombre des globules rouges est mesurée à l'aide d'une méthode de cytométrie avec analyse par diffusion de lumière laser optique et/ou d'une méthode d'impédance, l'activité AlAT est mesurée à l'aide d'une méthode spectrophotométrique cinétique ou d'une méthode calorimétrique, la concentration en peptide C est mesurée à l'aide d'une méthode ELISA, RIA ou d'une méthode de chimioluminescence, la concentration en SHBG est mesurée à l'aide d'une méthode IRMA, ELISA ou d'une méthode de chimioluminescence, la concentration en adiponectine est mesurée à l'aide d'une méthode RIA ou ELISA, et la concentration en IGFBP1 est mesurée à l'aide d'une méthode ELISA ou RIA.

**9.** Procédé ou utilisation selon la revendication 8, **caractérisé en ce que** le nombre des globules rouges est mesurée par une méthode de cytométrie avec analyse par diffusion de lumière laser optique, l'activité AlAT est mesurée par une méthode spectrophotométrique cinétique, la concentration en peptide C et IGFBP1 est mesurée par la méthode ELISA, la concentration en SHBG est mesurée par la méthode IRMA et la concentration en adiponectine est mesurée par la méthode RIA.

**10.** Procédé selon l'une quelconque des revendications 2 à 3 ou 8 à 9, ou utilisation selon l'une quelconque des revendications 4 à 9, **caractérisé en ce qu'un** trouble lié à la résistance à l'insuline est choisi dans un groupe comprenant : surpoids, obésité, intolérance au glucose, dyslipidémie, syndrome métabolique, stéatose hépatique, syndrome des ovaires polykystiques, hypertension artérielle, athérosclérose, cardiopathie ischémique, accident vasculaire cérébral, maladies neurodégénératives, en particulier diabète de type 2.

**11.** Procédé selon l'une quelconque des revendications 1 à 3 ou 8 à 10, ou utilisation selon l'une quelconque des revendications 4 à 10, **caractérisé en ce qu'un** sujet humain est choisi dans un groupe comprenant : une personne ayant un poids corporel normal, une personne en surpoids, une personne obèse, une personne ayant une glycémie à jeun élevée, une personne ayant une tolérance au glucose altérée, une personne atteinte de diabète de type 2, une personne atteinte de dyslipidémie, une personne atteinte du syndrome métabolique, une personne atteinte de stéatose hépatique, une femme atteinte du syndrome des ovaires polykystiques, une personne atteinte d'hypertension artérielle, une personne atteinte d'athérosclérose, une personne atteinte d'une cardiopathie ischémique, une personne après un accident vasculaire cérébral, une personne atteinte d'une maladie neurodégénérative, une femme ayant eu un diabète gestationnel ou ayant donné naissance à un bébé ayant un poids corporel supérieur à 4 kg, et un parent au premier degré d'une personne souffrant de diabète et/ou de maladies cardio-vasculaires, et une personne avant une chirurgie bariatrique et/ou après une telle chirurgie.

**Fig. 1**

ROC Curve
Slope coefficient = 1.46
Proposed cut-off poin: 7.519

**Fig. 2 A**

**Fig. 2 B**

Fig. 2 C

M (mg/kg FFM/min)

## REFERENCES CITED IN THE DESCRIPTION

**Non-patent literature cited in the description**

- **REAVEN GM.** Role of insulin resistance in human disease. *Diabetes*, 1988, vol. 37, 1595-1607
- **FERNANDEZ-REAL JM** ; **RICART W**. Insulin resistance and chronic cardiovascular inflammatory syndrome. *Endocr Rev*, 2003, vol. 24, 278-301
- **NIGRO J** ; **OSMAN N** ; **DART AM** ; **LITTLE PJ**. Insulin resistance and atherosclerosis. *Endocr Rev*, 2006, vol. 27, 242-259
- **RORIZ-FILHO J** ; **SA-RORIZ TM** ; **ROSSET I** ; **CAMOZZATO AL** ; **SANTOS AC** ; **CHAVES ML** ; **MORIGUTI JC** ; **RORIZ-CRUZ M.** Pre)diabetes, brain aging, and cognition. *Biochim Biophys Acta*, 2009, vol. 1792, 432-443
- **DEFRONZO RA** ; **TOBIN JD** ; **ANDRES R**. Glucose clamp technique: a method for quantifying insulin secretion and resistance. *Am J Physiol*, 1979, vol. 237, E214-E223
- **MUNIYAPPA R** ; **MADAN R** ; **QUON MJ**. Assessing insulin sensitivity and resistance in humans. MDText.com, Inc., 2000
- **ANTUNA-PUENTE B** ; **DISSE E** ; **RABASA-LHORET R** ; **LAVILLE M** ; **CAPEAU J** ; **BASTARD JP**. How can we measure insulin sensitivity/resistance?. *Diabetes Metab*, 2011, vol. 37 (9), 179-188
- **WALLACE TM** ; **MATTHEWS DR.** The assessment of insulin resistance in man.. *Diabet Med*, 2002, vol. 19, 527-534
- **FERRANNINI E** ; **MARI A**. How to measure insulin sensitivity. *J Hypertens*, 1998, vol. 16, 895-906
- **PATARRAO RS et al.** Assessment of methods and indexes of insulin sensitivity. *Rev Port Endocrinol Diabetes Metab*, 2014, vol. 9 (1), 65-73
- **MUNIYAPPA R et al.** Current approaches for assessing insulin sensitivity and resistance in vivo: advantages, limitations, and appropriate usage. *Am J Physiol Endocrinol Metab*, 2008, vol. 294 (1), E15-E26

- **MATSUDA M**. Measuring and estimating insulin resistance in clinical research setting.. *Nutr Metab Cardiovasc Dis*, 2010, vol. 20, 79-86
- **KO GT** ; **CHAN JC** ; **WOO J** ; **LAU E** ; **YEUNG VT** ; **CHOW CC** ; **COCKRAM CS**. The reproducibility and usefulness of the oral glucose tolerance test in screening for diabetes and other cardiovascular risk factors.. *Ann Clin Biochem*, 1998, vol. 35, 62-67
- **LIBMAN IM** ; **BARINAS-MITCHELL E** ; **BARTUCCI A** ; **ROBERTSON R** ; **ARSLANIAN S**. Reproducibility of the Oral Glucose Tolerance Test in Overweight Children. *J Clin Endocrinol Metab*, 2008, vol. 93, 4231-4237
- **RUIGE JB** ; **MERTENS IL** ; **BARTHOLOMEEUSEN E** ; **DIRINCK E** ; **FERRANNINI E** ; **VAN GAAL LF.** Fasting-based estimates of insulin sensitivity in overweight and obesity: a critical appraisal. *Obesity*, 2006, vol. 14, 1250-1256
- **MALITA FM** ; **KARELIS AD** ; **ST-PIERRE DH** ; **GARREL D** ; **BASTARD JP** ; **TARDIF A** ; **PRUD'-HOMME D** ; **RABASA-LHORET R.** Surrogate indexes vs. euglycaemic-hyperinsulinemic clamp as an indicator of insulin resistance and cardiovascular risk factors in overweight and obese postmenopausal women. *Diabetes Metab*, 2006, vol. 32, 251-255
- **RORIZ-FILHO, J. et al.** *Biochim Biophys Acta*, 2009, vol. 1792, 432-443
- **HASTIE T** ; **TIBSHIRANI T** ; **NARASIMHAN B** ; **CHU G**. *Imputation for microarray data*, 2014
- **FRIEDMAN J** ; **HASTIE T** ; **TIBSHIRANI R.** Regularization Paths for Generalized Linear Models via Coordinate Descent. *Journal of Statistical Software*, 2010, vol. 33, 1-22, http://www.jstatsoft.org/-v33/i01
- **BAIR E.** ; **TIBSHIRANI R**. *Supervised principal components*, 2012, http://CRAN.R-project.org/package=superpc